# FASCICULE DE BREVET EUROPEEN

(11) **EP 2 387 434 B1**
(45) Date de publication et mention de la délivrance du brevet: **28.10.2015**
(21) Numéro de dépôt: 09797076.8
(22) Date de dépôt: 24.11.2009
(51) Int. Cl.: A61M 39/28, A61B 17/122

(54) **SECTIONNEMENT D'UN TUBE SOUPLE DESTINE A DES APPLICATIONS BIOPHARMACEUTIQUES**
SCHNEIDEN EINES FLEXIBLEN SCHLAUCHS ZUR BIOPHARMAZEUTISCHEN VERWENDUNG
CUTTING A FLEXIBLE TUBE FOR BIOPHARMACEUTICAL USE

(30) Priorité: 10.12.2008 FR 0806951
(43) Date de publication de la demande: 23.11.2011
(73) Titulaire: SARTORIUS STEDIM FMT SAS, 13400 Aubagne (FR)
(72) Inventeur: GAY, Isabelle, Valérie, Christine, F-13124 Peypin (FR); BATES, Michael, Gloucester, Gloucestershire GL3 4PD (GB); BASSETTS, Ray, Stroud, Glos Gloucestershire GL5 4NY (GB); BIDDEL, Chris, Gloucestershire Gloucesterhire GL10 3LH (GB)
(74) Mandataire: Derambure, Christian
(86) Numéro de dépôt international: PCT/FR2009/052283
(87) Numéro de publication internationale: WO 2010/066977

(56) Documents cités:
- EP-A- 0 799 627
- WO-A-2005/117723
- FR-A- 1 534 916
- FR-A- 2 323 939
- FR-A- 2 492 261
- US-A- 4 688 753
- US-A- 5 676 672

## Description

L'invention concerne le sectionnement d'un tube souple tel que typiquement destiné à des applications biopharmaceutiques. Elle vise plus spécialement un procédé pour fermer et sectionner un tube souple dans une région de pincement, un dispositif de pincement de fermeture pour le sectionnement et un organe de coupe, spécialement destinés à coopérer et former un ensemble de pincement de fermeture et de sectionnement apte à la mise en oeuvre du procédé.

Un tube tel que celui auquel s'applique l'invention permet la circulation, le passage, la communication d'un fluide, tel qu'un fluide biopharmaceutique. Par exemple, un tel tube est interposé entre deux - ou un plus grand nombre de - poches.

Un tel tube, habituellement de section circulaire, est réalisé typiquement en matière plastique telle que le silicone, mais également le PVC, la liste n'étant pas limitative. Il présente une certaine tenue d'ensemble et, simultanément, à la fois une certaine flexibilité d'ensemble et une certaine flexibilité locale, ce qui permet, moyennant l'exercice d'une force suffisante de pincement, de pincer le tube en l'aplatissant et en l'écrasant jusqu'à le fermer sur lui-même.

Dans une réalisation typique, par exemple, le tube a un diamètre extérieur compris entre par exemple 8 millimètres et 30 millimètres, l'épaisseur dépendant du matériau, du diamètre et des applications.

Le plus souvent, un tel tube soit fait partie d'un dispositif comportant une ou plusieurs poches ou des moyens fonctionnels (filtration, mélange...), soit est monté sur, et associé fonctionnellement à, un appareil ou dispositif de traitement (prélèvement, circulation...).

Le plus souvent, les opérations portant sur de tels tubes et notamment le sectionnement doivent être aseptiques, c'est-à-dire que l'intégrité bactérienne ne doit pas être remise en cause.

Dans un certain nombre de cas, le processus concernant le fluide canalisé par le tube nécessite à un moment ou un autre que le tube soit fermé et sectionné dans une région donnée afin d'empêcher la circulation, le passage, la communication du fluide de part et d'autre de cette région et de pouvoir disposer séparément des deux portions du tube ainsi fermé et sectionné et ce qui lui est attenant, tel qu'une poche, un filtre ou autre moyen fonctionnel.

Le document US 5 925 052 décrit un dispositif de sectionnement et de pincement des parties extrêmes sectionnées d'un cordon ombilical, comprenant un outil à main formant pince agencée pour être manipulé par un opérateur, destiné à recevoir de façon amovible un ensemble unitaire incluant une paire de supports de pinces et une surface de coupe placée entre eux. La paire de supports de pinces est destinée à son tour à recevoir deux pinces distinctes séparées, dont les mors comportent des empreintes pourvues de dents s'inscrivant dans une enveloppe plate. Entre les deux mors des deux pinces, peut être placé le cordon ombilical à sectionner. Ces deux mors sont pourvus de moyens de verrouillage tels qu'une fois les mors fermés, ils le restent. Le premier mors de chaque pince est destiné à reposer sur le support de pince correspondant, lui-même destiné à reposer sur le premier mors de l'outil à main. Le second mors de chaque pince, articulé sur le premier mors, est déplacé par un tourillon porté par le second mors de l'outil à main, lequel est pourvu d'une lame de coupe destinée à coopérer avec la surface de coupe de l'ensemble unitaire incluant une paire de supports de pinces, et passant à cet effet entre les deux seconds mors des deux pinces.

Un tel dispositif a plusieurs limitations et inconvénients. Tout d'abord, il est spécialement adapté au sectionnement d'un cordon ombilical et non à un tube tel que celui ici concerné. Ensuite, il requiert un outil à main formant pince ayant une poignée jouant un rôle de levier pour les mors. Le pincement et le sectionnement sont nécessairement concomitants. L'usage de pinces à charnière ne permet pas une compression uniforme du cordon ombilical. Le pincement est obtenu par une compression à plat qui, le plus souvent, est source de fuite. Le dispositif n'est pas vraiment adapté à des cordons ombilicaux de différentes géométries. Les deux pinces et les deux supports de pinces sont nettement écartés les uns des autres afin de permettre le logement entre eux de la surface de coupe, laquelle est située dans le même plan que l'enveloppe des empreintes des mors situés du même côté. Il s'ensuit que la coupe du cordon ombilical ne peut pas être faite d'un côté à l'autre des deux pinces.

Le document EP-A-0 799 627 décrit un dispositif de pincement et de sectionnement dérivé des pinces utilisées dans le domaine biopharmaceutique et connues sous le nom de pinces Halkey ou de pinces Roberts ou encore de pinces Halkey-Roberts. Le dispositif associe à une telle pince une lame de coupe. Un tel dispositif présente les mêmes inconvénients que ceux mentionnés en relation avec le document US 5 925 052.

Le document US 7 410 155 décrit un procédé pour pincer et couper un tube déformable destiné à des applications biopharmaceutiques et un dispositif pour pincer un tel tube. Selon le procédé décrit dans ce document, on dispose le tube entre les première et seconde sections articulées entre elles d'un dispositif de pincement, chaque section ayant deux parties articulées entre elles. Puis, par un mouvement rotatif relatif de fermeture des première et seconde sections, on les ferme l'une sur l'autre et on pince le tube en l'aplatissant entre les deux paires de parties du dispositif de pincement. On fait coopérer des moyens de verrouillage réciproque des deux parties de chaque paire de parties des première et seconde sections pour les maintenir fermés et maintenir le tube pincé. Puis, on coupe le tube entre les deux paires de parties du dispositif et au moins une partie des première et seconde section, afin de former deux longueurs séparées de tube pincées chacune à sa partie extrême coupée. Grâce à la coopération des moyens de verrouillage, on maintient au moins temporairement chaque paire de parties du dispositif de pincement pincée sur la longueur correspondante du tube, une fois le dispositif de pincement coupé en deux parties. Un tel dispositif de pincement comporte une première section et une seconde section articulées entre elles, chaque section ayant elle-même deux parties articulées entre elles. La première et de la seconde section sont agencées de manière à pouvoir être déplacées à pivotement de façon relative l'une par rapport à l'autre entre une position ouverte et une position fermée. Les quatre parties comportent une empreinte en creux à fond plat venant sur le tube de part et d'autre pour le pincer. Le dispositif comporte également des moyens de verrouillage réciproque des deux parties de chaque paire de parties des deux sections pour les maintenir au moins temporairement en position fermée. Un tel dispositif est destiné à être sectionné pour pouvoir sectionner le tube.

Un tel procédé et un tel dispositif présentent nombre d'inconvénients qui se combinent les uns aux autres. Comme précédemment, l'usage de charnières ne permet pas une compression uniforme du tube. Le pincement est obtenu par une compression à plat avec le risque de fuite inhérent. Le dispositif n'est pas adapté à des tubes de différentes géométries (diamètre et/ou épaisseur). Le sectionnement du tube nécessite celui du dispositif, ce qui est une opération par ailleurs inutile mais pourtant source d'inconvénients, ce sectionnement pouvant générer des petits morceaux gênants du matériau constitutif du dispositif.

Les documents US 5 676 672 et WO 2005/117723 décrivent des dispositifs de pincement et de sectionnement d'un cordon ombilical, dans lesquels une lame de coupe vient frapper une partie du dispositif sur laquelle repose le cordon ombilical. De tels dispositifs ont également plusieurs limitations et inconvénients. Ils sont spécialement adaptés au sectionnement d'un cordon ombilical et non à un tube tel que celui ici concerné. La présence de charnières ne permet pas une compression uniforme du cordon ombilical. Le pincement est obtenu par une compression à plat, source de fuite.

L'invention vise à remédier aux limitations et inconvénients des procédés et dispositifs connus. Elle vise à permettre le pincement de fermeture et le sectionnement d'un tube souple destiné à des applications biopharmaceutiques. Elle a pour but de s'affranchir de la nécessité d'un outil à main tel qu'une pince. Elle vise à permettre une opération de sectionnement ultérieure au pincement. Elle vise également une compression du tube à sectionner qui soit uniforme et à un pincement sans risque de fuite et adapté à des tubes de différentes géométries. Elle vise également à ce que la coupe du tube puisse être faite d'un côté à l'autre. Elle vise également à éviter de devoir sectionner le dispositif lui-même et donc à éviter les petits morceaux gênants du matériau constitutif du dispositif. Elle vise à permettre au tube une fois sectionné d'être fermé de façon permanente, et ce en utilisant le même ensemble de pincement de fermeture et de sectionnement. Elle vise à ce que cet ensemble de pincement de fermeture et de sectionnement soit à usage unique et remplisse une fonction de fermeture permanente du tube sur lui-même dans la région de pincement empêchant tout passage du fluide dans le tube, ce que les dispositifs selon les documents de l'état de la technique ne permettent pas. En effet, l'exigence d'une fermeture empêchant tout passage du fluide dans le tube est d'autant plus important que la fermeture est permanente, en tout cas n'a pas vocation à être réversible, cette exigence étant moins cruciale dans le cas d'une fermeture temporaire et réversible.

L'invention vise à proposer un tel ensemble de pincement de fermeture et de sectionnement qui soit à la fois peu coûteux, aisé à fabriquer, simple et sûr à utiliser. En effet, un tel dispositif doit pouvoir être mis en place sans difficulté mais de manière efficace dans le cours du processus concernant le fluide canalisé par le tube.

A cet effet, selon un premier aspect, l'invention a pour objet un procédé pour sectionner et fermer un tube souple du type dans lequel :
- on dispose du tube à sectionner et à fermer,
- on dispose d'un dispositif de pincement de fermeture pour le sectionnement et d'au moins un organe de coupe, le dispositif de pincement de fermeture pour le sectionnement comprenant deux sections, chacune ayant deux parties creuses, une couple au moins étant à empreintes, ces parties creuses pouvant être placées dans le prolongement l'une de l'autre par des moyens d'association tout en ménageant entre elles un passage transversal de sectionnement ayant un accès apte au passage de l'organe de coupe ou d'un organe de commande de l'organe de coupe, le dispositif de pincement de fermeture pour le sectionnement pouvant se trouver soit à l'état ouvert où les deux sections sont écartées l'une de l'autre soit à l'état fermé où elles sont rapprochées et fermées l'une sur l'autre et maintenues fermées par des moyens de verrouillage et où les faces des empreintes forment un passage de pincement et de fermeture du tube,

- on dispose du dispositif de pincement de fermeture pour le sectionnement à l'état ouvert,
- on positionne une région de pincement du tube dans l'empreinte d'une section et on amène l'autre section en regard de la précédente et le dispositif de pincement de fermeture pour le sectionnement à l'état fermé, avec coopération des moyens de verrouillage, jusqu'à ce que le tube soit positivement pincé et fermé dans au moins une zone de pincement positif de fermeture par les faces des empreintes, et on maintient le tube ainsi pincé et fermé entre les sections,
- on sectionne le tube entre les parties du dispositif de pincement de fermeture pour le sectionnement à l'état fermé, à l'aide du au moins un organe de coupe,
- on dissocie les moyens d'association et donc les parties du dispositif de pincement de fermeture pour le sectionnement, dont la au moins une couple de parties à empreintes reste fixée à la partie extrême sectionnée du tube en la pinçant et en la fermant.

Ce procédé est tel que :
- on dispose d'un dispositif de pincement de fermeture pour le sectionnement dont l'une des empreintes est une empreinte femelle, l'autre des empreintes est une empreinte mâle, les deux empreintes, à l'état fermé du dispositif ménageant entre elles un passage de pincement positif de fermeture limité par les deux faces des empreintes, ayant, en section droite transversale, une forme générale de U,
- et, pour sectionner le tube, on fait passer le au moins un organe de coupe dans le passage de sectionnement sans que l'organe de coupe ne sectionne, ne pénètre dans, ou n'interfère frontalement avec le dispositif de pincement de fermeture pour le sectionnement.

Selon une possibilité, on sectionne le tube immédiatement après ou un certain temps après que l'on a réalisé son pincement positif de fermeture.

Selon une possibilité, on fait passer l'organe de coupe dans le passage de sectionnement sans contact avec le dispositif de pincement de fermeture pour le sectionnement. Selon une autre possibilité, on fait passer l'organe de coupe dans le passage de sectionnement avec un contact surfacique glissant avec le dispositif de pincement de fermeture pour le sectionnement.

Selon les cas, on sectionne le tube au moyen d'un seul organe de coupe ou de deux organes de coupe opposés.

Selon une possibilité, on sectionne le tube dans une zone d'aplatissement dans laquelle on ne pince pas positivement le tube.

Selon une possibilité, on amène une section en regard d'une autre et le dispositif de pincement de fermeture pour le sectionnement à l'état fermé, par un mouvement relatif de translation transversale ou de pivotement autour d'un axe qui, par rapport à l'axe longitudinal du dispositif est déporté sur un côté longitudinal du dispositif.

Selon une possibilité, on maintient le tube positivement pincé et fermé dans une seule zone de pincement positif de fermeture et on maintient le tube dans l'autre zone. Selon une autre possibilité, on maintient le tube positivement pincé et fermé dans deux zones de pincement positif de fermeture.

Selon une première possibilité, on dissocie les moyens d'association et donc les parties du_ dispositif de pincement et de fermeture, alors que l'une seulement des couples de parties à empreintes reste fixée à la partie extrême sectionnée du tube en la pinçant et en la fermant. Selon une seconde possibilité, on dissocie les moyens d'association alors que les deux couples de parties à empreintes restent fixées à la partie extrême sectionnée du tube en la pinçant et en la fermant.

Selon une possibilité, on commence le pincement de fermeture du tube dans la zone des deux plis latéraux du tube.

Selon un deuxième aspect, l'invention a pour objet un dispositif de pincement de fermeture pour le sectionnement spécialement destiné, en combinaison avec un organe de coupe, à la mise en oeuvre du procédé qui vient d'être décrit, qui comprend deux sections, chacune ayant deux parties creuses, une couple au moins étant à empreintes, ces parties pouvant être placées dans le prolongement l'une de l'autre de façon amovible par des moyens d'association tout en ménageant entre elles un passage transversal de sectionnement ayant un accès apte au passage de l'organe de coupe ou d'un organe de commande de l'organe de coupe, le dispositif de pincement de fermeture pour le sectionnement pouvant se trouver soit à l'état ouvert où les deux sections sont écartées l'une de l'autre soit à l'état fermé où elles sont rapprochées et fermées l'une sur l'autre et maintenues fermées par des moyens de verrouillage et où les faces des empreintes forment un passage de pincement et de fermeture du tube comportant au moins un tronçon auquel est adjacent axialement un espace dans lequel débouche le passage et où le tube est d'une part maintenu et d'autre part aplati avec un certain degré, l'une des empreintes étant une empreinte femelle, l'autre des empreintes étant une empreinte mâle, les deux empreintes, à l'état fermé du dispositif de pincement de fermeture pour le sectionnement ménageant entre elles un passage de pincement positif de fermeture limité par les deux faces des empreintes, ayant, en section droite transversale, une forme générale de U.

Selon une réalisation, la largeur de l'ouverture du passage de pincement, définie par l'écartement transversal entre les faces de section droite transversale en forme générale de U, est légèrement plus petite que le double de l'épaisseur de la paroi du tube souple à pincer.

Selon une réalisation, l'empreinte mâle a en section droite transversale une forme générale de U avec une âme et deux ailes, l'empreinte femelle a en section droite transversale une forme générale de U avec une âme et deux ailes et le passage de pincement a en section droite transversale une forme générale de U avec une âme et deux ailes ; les deux ailes en regard de chaque couple d'ailes en regard de l'empreinte mâle et de l'empreinte femelle vont en se rapprochant l'une de l'autre vers leurs extrémités opposées aux âmes des empreintes, au moins dans la zone de ces extrémités et chacune des deux ailes du passage de pincement a, entre les faces des deux empreintes une ouverture dont la largeur va en diminuant vers son extrémité opposée à l'âme du passage de pincement.

Selon une réalisation, les sections sont profilées et s'étendent le long de l'axe longitudinal, la zone de pincement positif de fermeture s'étendant sur une certaine longueur axiale.

Selon une réalisation, les deux parties creuses de chaque section sont analogues, le dispositif de pincement de fermeture pour le sectionnement assurant une fonction de fermeture du tube sur lui-même dans deux zones de pincement positif de fermeture. Dans ce cas, le passage de pincement positif de fermeture est en deux tronçons de pincement positif de fermeture placés dans le prolongement l'un de l'autre avec un écartement entre eux où le passage de pincement positif de fermeture est absent pour ne pas exister.

Selon une autre réalisation, les deux parties creuses de chaque section sont différentes, le dispositif de pincement de fermeture pour le sectionnement assurant une fonction de fermeture du tube sur lui-même dans une seule zone de pincement positif de fermeture et étant seulement maintenu dans l'autre zone.

Selon une réalisation, les moyens de verrouillage sont irréversibles pour les deux couples de parties creuses. Selon une autre réalisation, les moyens de verrouillage sont irréversibles pour l'une des couples de parties creuses et réversibles pour l'autre. En particulier, les moyens de verrouillage sont réversibles pour une couple de parties creuses n'assurant qu'un simple maintien du tube T.

Selon une réalisation, les moyens de verrouillage sont irréversibles, tels que les deux sections amenées à l'état fermé sont maintenues dans cet état par les moyens de verrouillage sans pouvoir être dissociées, ces moyens de verrouillage comprenant des dents d'accrochage profilées, orientées selon deux directions opposées.

Selon une réalisation, le passage de pincement est d'ouverture réglable, le mouvement relatif pour amener une section en regard d'une autre et le dispositif de pincement de fermeture pour le sectionnement à l'état fermé étant plus ou moins grand, pour que le passage de pincement soit respectivement, plus ou moins petit.

Selon une réalisation, les deux empreintes ont une même forme, une même disposition et de mêmes dimensions, constantes et uniformes, d'une extrémité libre à l'autre. Selon une autre réalisation, l'une ou/et l'autre des deux empreintes est légèrement inclinée le long de l'axe du dispositif de pincement de fermeture pour le sectionnement, vers le bord libre de chant de la rive et vers l'extrémité proximale, pour exercer un pincement du tube plus important vers l'extrémité proximale et expulser le fluide se trouvant dans le tube vers l'extrémité distale.

Selon une réalisation, le passage transversal de sectionnement est ménagé entre les deux couples de faces transversales d'extrémité et proximales des sections, situées en regard l'une de l'autre et écartées à proximité l'une de l'autre le long de l'axe du dispositif de pincement de fermeture pour le sectionnement.

Selon une réalisation, il est prévu des moyens d'écartement constitués par des butées ménagés sur les faces d'extrémité et proximales ou les moyens d'association.

Selon une réalisation, les moyens d'association comprennent au moins une saillie ménagé sur l'une des parties du dispositif, apte à coopérer de façon amovible avec au moins un trou borgne ou creux ou rainure complémentaire, ménagé sur l'autre partie de la même section. Par exemple, une saillie et un trou ou rainure s'étend parallèlement à l'axe ou transversalement.

Selon une autre réalisation, les moyens d'association comprennent au moins une pièce amovible de liaison en forme de clip, apte à associer de façon amovible les deux parties de façon indirecte, le clip comprenant une âme de laquelle sont attenants quatre saillies en forme de tige aptes à coopérer avec quatre rainures transversales ménagées sur la face extérieure du dispositif de pincement de fermeture pour le sectionnement, sur chaque partie.

Selon une autre réalisation, les moyens d'association comprennent une pièce amovible de liaison en forme de berceau, apte à associer de façon amovible les deux parties de façon indirecte, le berceau comportant deux rives se faisant face, aptes à venir enserrer les parties par leurs faces extrêmes distales.

Selon une première réalisation, les deux sections sont deux pièces distinctes et sont amenées à l'état fermé par un mouvement relatif de coulissement transversal.

Dans cette réalisation, l'une des deux sections est une section mâle et l'autre une section femelle, la section mâle comportant et étant limitée extérieurement par une première âme et deux premières rives, la section femelle comportant et étant limitée extérieurement par une seconde âme et deux secondes rives.

Dans cette réalisation, l'écartement entre les deux faces extérieures opposées des deux premières rives est en correspondance avec l'écartement entre les deux faces intérieures en regard des deux secondes rives, de manière à permettre, à l'état fermé du dispositif de pincement de fermeture pour le sectionnement, l'emboîtement avec ajustement des deux sections, la couple de faces extérieures étant alors en contact et en appui sur la couple de faces intérieures.

Selon une réalisation, les moyens de verrouillage réciproque sont deux ensembles comprenant une série de dents ménagées respectivement sur les deux faces extérieures des premières rives et les deux faces intérieures des secondes rives dans leur zone d'emboîtement.

Selon une réalisation, la section mâle définit une empreinte femelle et la section femelle définit une empreinte mâle.

Selon une seconde réalisation, les deux sections sont deux pièces associées structurellement l'une à l'autre à pivotement autour d'un axe qui, par rapport à l'axe longitudinal du dispositif de pincement de fermeture pour le sectionnement est déporté sur un côté longitudinal du dispositif, les deux sections étant amenées à l'état fermé par un mouvement relatif de pivotement autour de l'axe de pivotement.

Dans cette réalisation, les moyens de verrouillage réciproque comprennent des dents situées du côté opposé à l'axe de pivotement. Plus précisément, les moyens de verrouillage réciproque comprennent une pièce femelle rapportée sur l'une des parties et pourvue de dents et une pièce mâle rapportée sur l'autre des parties et pourvue de dents.

Selon une réalisation, les deux sections sont de même longueur axiale.

Selon un troisième aspect, l'invention a pour objet un organe de coupe spécialement destiné, en combinaison avec un dispositif de pincement de fermeture pour le sectionnement à la mise en oeuvre du procédé décrit plus haut, tel que sa forme et ses dimensions sont choisies de manière que l'organe de coupe puisse via un accès passer dans un passage transversal de sectionnement du dispositif de pincement de fermeture pour le sectionnement et ainsi sectionner le tube pincé et fermé par ce dispositif.

Selon un quatrième aspect, l'invention a pour objet un ensemble de pincement de fermeture et de sectionnement comprenant un dispositif de pincement de fermeture pour le sectionnement tel que décrit précédemment et au moins un organe de coupe tel que décrit précédemment, l'organe de coupe étant apte à passer dans le passage de sectionnement sans qu'il ne sectionne, ne pénètre dans, ou n'interfère frontalement avec le dispositif de pincement de fermeture pour le sectionnement.

Selon une première réalisation d'un tel ensemble , le dispositif de pincement de fermeture pour le sectionnement inclut structurellement et fonctionnellement le au moins un organe de coupe.

Dans cette réalisation, l'organe de coupe peut être monté coulissant dans son propre plan sur la face d'extrémité proximale de l'une des parties.

Selon une seconde réalisation, le dispositif de pincement de fermeture pour le sectionnement n'inclut pas structurellement le au moins un organe de coupe, celui-ci lui étant associé fonctionnellement.

On décrit maintenant plusieurs modes de réalisation de l'invention à l'aide des dessins dans lesquels :
- la figure 1 est une vue en perspective d'une première famille de réalisation possible d'un ensemble de pincement de fermeture et de sectionnement à l'état ouvert, avec une portion de tube en position, un organe de coupe étant représenté schématiquement,
- la figure 2 est une vue en coupe axiale, partielle, à plus grande échelle d'un ensemble de pincement de fermeture et de sectionnement et d'une portion de tube en position, juste avant le début du sectionnement du tube,
- la figure 3 est une vue en coupe transversale d'un ensemble de pincement de fermeture et de sectionnement à l'état fermé, avec une portion de tube en position,
- la figure 3A est une vue partielle à plus grande échelle de la figure 3,
- la figure 4 est une vue en coupe longitudinale d'une réalisation possible d'un ensemble de pincement de fermeture et de sectionnement à l'état fermé, avec une portion de tube en position,
- les figures 5, 6 et 7 sont trois vues en perspective de trois réalisations possibles de moyens de verrouillage d'un ensemble de pincement de fermeture et de sectionnement représenté partiellement,
- la figure 8 est une vue en perspective d'une autre réalisation possible de l'organe de coupe associé à un ensemble de pincement de fermeture et de sectionnement représenté partiellement,
- les figures 9 et 10 sont deux vues en perspective d'une seconde famille de réalisation possible d'un ensemble de pincement de fermeture et de sectionnement.

Un ensemble de pincement de fermeture et de sectionnement comprend un dispositif 1 qui permet d'assurer un pincement de fermeture d'un tube T tel que précédemment défini, dans la région souhaitée, combiné fonctionnellement avec au moins un organe de coupe 2, intégré ou extérieur à lui qui permet d'assurer le sectionnement transversal du tube T.

Une fois que le dispositif 1 de pincement de fermeture pour le sectionnement a été mis en oeuvre pour assurer le pincement positif de fermeture du tube T et son sectionnement transversal au moyen de l'organe de coupe 2, le dispositif 1 de pincement de fermeture pour le sectionnement a vocation à rester fixé sur le tube T.

L'ensemble 1 + 2, comme le dispositif 1 de pincement de fermeture pour le sectionnement, peut être qualifié d'ensemble, ou de dispositif, à usage unique.

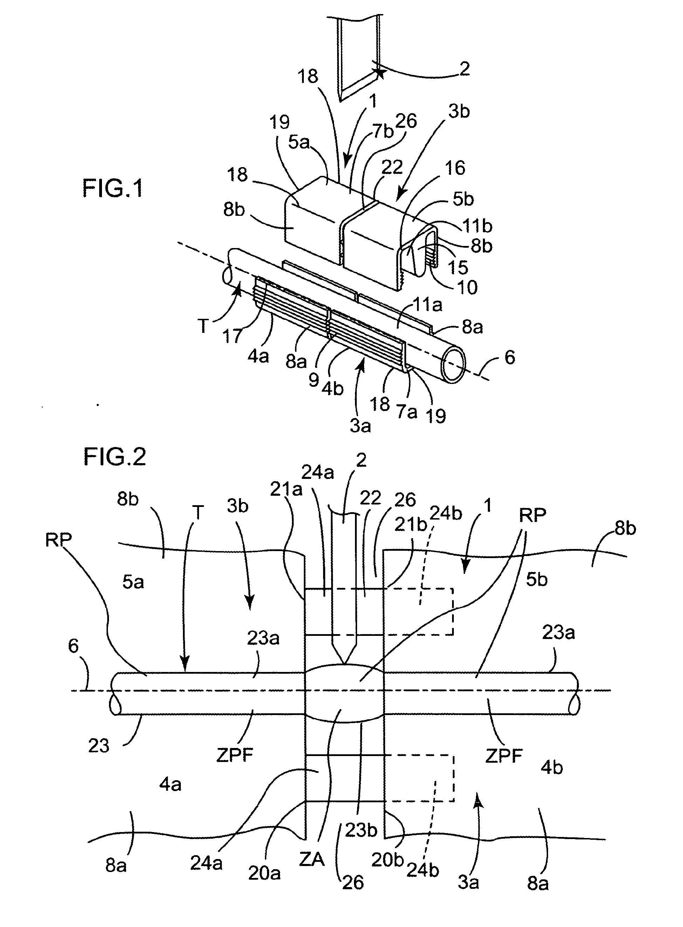

La figure 1 illustre un ensemble comprenant un dispositif 1 de pincement de fermeture pour le sectionnement incluant deux sections 3, creuses, profilées, à savoir une première section mâle 3a et une seconde section femelle 3b.

Chaque section 3a, 3b comporte deux parties également creuses et profilées, à savoir, respectivement, les deux parties 4a et 4b pour la section mâle 3a et les deux parties 5a et 5b pour la section femelle 3b.

Les deux sections 3a et 3b sont aptes, en fonction des phases opératoires, à être structurellement et fonctionnellement associées entre elles par un emboîtement normalement irréversible, pour former un ensemble rigide, les deux parties creuses étant en regard (figure 2), (phase de pincement, phase de sectionnement) ou à être dissociées (figure 1) (phase de mise en place).

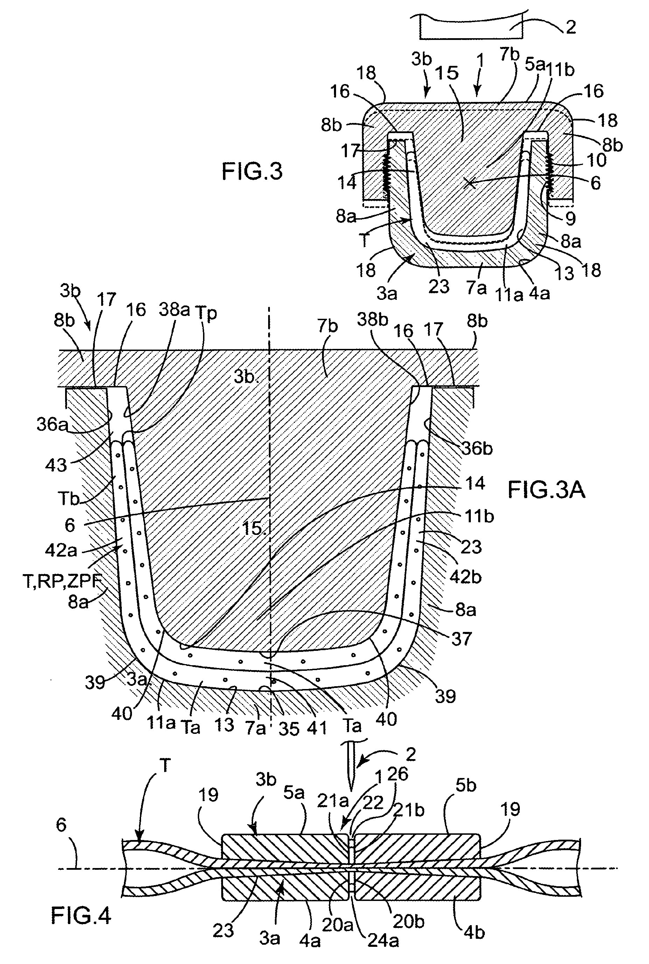

Les deux parties 4a et 4b ou 5a et 5b d'une même section 3a ou 3b sont aptes, selon les phases opératoires, à être structurellement et fonctionnellement associées entre elles en étant placées dans le prolongement l'une de l'autre (figure 1) (phase de pincement, phase de sectionnement), ou à être dissociées (figures 4, 5 et 6) (phase de dissociation).

Lorsque les deux sections 3a et 3b sont structurellement et fonctionnellement associées entre elles par emboîtement, leurs parties en regard respectives 4a et 5a d'une part, 4b et 5b d'autre part sont également structurellement et fonctionnellement associées entre elles par un emboîtement qui, dans les conditions normales d'utilisation est irréversible.

Les sections sont référencées de façon générique 3 et les parties 4 et 5.

Le dispositif 1 de pincement de fermeture pour le sectionnement comprend donc les quatre pièces distinctes 4a, 4b, 5a et 5b, compactes, profilées, rigides et typiquement réalisées en matière plastique telle qu'un polymère choisi pour satisfaire, le cas échéant, les autres contraintes auxquelles le dispositif 1 de pincement de fermeture pour le sectionnement doit faire face, par exemple, résister à un traitement en autoclave ou par rayonnement γ.

Dans une réalisation, les parties 4a et 4b de la section 3a sont, sinon strictement identiques, du moins analogues entre elles. De même en ce qui concerne les parties 5a et 5b de la section 3b. Dans ce cas, le dispositif 1 de pincement de fermeture pour le sectionnement assure une fonction de fermeture du tube T sur lui-même dans deux zones de pincement positif de fermeture.

Mais, dans une autre réalisation, les deux parties creuses 4a, 4b et 5a, 5b ne sont pas analogues, mais différentes, de sorte que, par exemple, le dispositif 1 de pincement de fermeture pour le sectionnement n'assure une fonction de fermeture du tube T sur lui-même que dans une seule zone de pincement positif de fermeture. Dans son autre zone, le tube T est alors simplement maintenu, ce qui facilite son sectionnement transversal. Le cas échéant, ce maintien est amovible, n"existant que tant que nécessaire pour le sectionnement.

Le dispositif 1 de pincement de fermeture pour le sectionnement présente un axe longitudinal 6 qui est également l'axe longitudinal du tube T lorsque celui-ci est placé dans le dispositif 1 de pincement de fermeture pour le sectionnement. C'est en relation avec cet axe 6 qu'est qualifié de « axial » toute direction ou plan s'étendant parallèlement à l'axe 6 et de « transversal » toute direction ou plan s'étendant orthogonalement à l'axe 6.

Les deux sections 3a et 3b sont profilées et s'étendent le long de l'axe 6.

Dans la réalisation représentée, les deux sections 3a et 3b sont monobloc et de même longueur axiale, de manière à pouvoir être emboîtées en étant ajustées, en regard l'une de l'autre.

Chacune des deux sections 3a et 3b, respectivement chacune de ses deux parties 4a, 4b, et 5a, 5b, a un contour extérieur ayant en section droite transversale une forme générale de. U. Ce contour comporte, et est limité par, plusieurs tronçons, à savoir une âme et, de part et d'autre de celle-ci, deux rives latérales, l'âme et les deux rives s'étendant par ailleurs axialement.

On entend par « forme générale de U », une forme définie par une âme médiane et deux ailes latérales de part et d'autre, en regard l'une de l'autre et s'étendant dans une même direction générale commune.

La première section mâle 3a comporte, et est limitée extérieurement par, une première âme 7a et deux premières rives 8a. La seconde section femelle 3b comporte, et est limitée extérieurement par, une seconde âme 7b et deux secondes rives 8b.

L'écartement entre les deux faces extérieures 9 opposées des deux premières rives 8a est en correspondance avec l'écartement entre les deux faces intérieures 10 en regard des deux secondes rives 8b. Cette disposition permet, à l'état fermé du dispositif 1 de pincement de fermeture pour le sectionnement, l'emboîtement avec ajustement des deux sections 3a et 3b, respectivement de leurs parties constitutives, la couple de faces extérieures 9 étant alors en contact et en appui sur la couple de faces intérieures 10.

Dans la réalisation représentée, la première section mâle 3a définit une première empreinte femelle 11 a et la seconde section femelle 3b définit une seconde empreinte mâle 11 b.

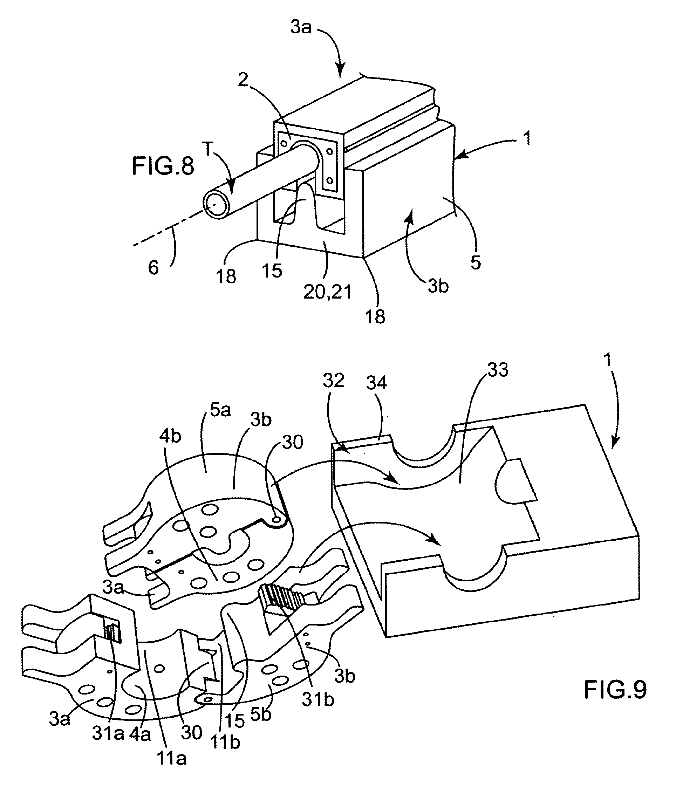

Selon une autre réalisation, la disposition est inverse, la première section mâle 3a définissant une première empreinte mâle et la seconde section femelle 3b définissant une seconde empreinte femelle (figure 8).

La première empreinte femelle 11a est définie par une face 13 profilée, s'étendant le long de l'axe 6, ayant en section transversale un contour de l'intrados d'un U et s'étendant parallèlement à l'axe 6. La face 13 est constituée de la face intérieure 35 de la première âme 7a, disposée de façon médiane, et des deux faces intérieures 36a et 36b des deux premières rives 8a, disposées latéralement de part et d'autre de la face intérieure 35. Ainsi, l'empreinte femelle 11 a comporte une âme 35 et deux ailes 36a et 36b.

La seconde empreinte mâle 11 b est définie par une face 14 profilée, s'étendant le long de l'axe 6, ayant en section transversale un contour de l'extrados d'un U et s'étendant parallèlement à l'axe 6. La face 14 est constituée de la face extérieure 37 de la partie d'extrémité d'une saillie 15, disposée de façon médiane, et des deux faces extérieures 38a et 38b du corps de la saillie 15, placées de part et d'autre de la face extérieure 37. Ainsi, l'empreinte mâle 11 b comporte une âme 37 et deux ailes 38a et 38b.

La saillie 15 est attenante à la seconde âme 7b et s'étend à égale distance entre les deux secondes rives 8b en étant dirigée dans le même sens qu'elles.

La face 14 est reliée aux deux faces intérieures 10 des deux rives 8b par deux petites faces sensiblement coplanaires 16. Ces deux petites faces 16 sont susceptibles, le cas échéant, lorsque l'emboîtement des deux sections 3a et 3b est le plus grand, d'être en contact et en appui sur les deux chants libres 17 des deux rives 8a, à l'état fermé du dispositif 1 de pincement de fermeture pour le sectionnement.

Bien entendu, pour pouvoir réaliser l'emboîtement requis, les deux faces 13 et 14 sont disposées tête-bêche l'une par rapport à l'autre. Si l'une est disposée en U (U droit), l'autre est disposée en ∩ (∪ inversé).

Les faces 13 et 14 sont conjuguées de manière à pouvoir coopérer entre elles et assurer le pincement du tube T sur lui-même jusqu'à sa fermeture totale, les âmes 35 et 36 étant conjuguées entre elles, de même que d'une part les ailes 36a et 38a, d'autre part les ailes 36b et 38b. Comme les empreintes 11a, 11b, le pincement s'étend le long de l'axe 6, c'est-à-dire qu'il n'est pas ponctuel, mais linéaire sur une certaine longueur.

Les faces 13 et 14 sont globalement lisses. Dans la réalisation représentée, les faces 13 et 14 ont des formes simples et non pas des formes tourmentées.

Dans la réalisation représentée, les âmes 35 et 37 ont une forme globalement aplanie avec une légère incurvation, respectivement concave et convexe. Les ailes 36a, 36b, 38a, 38b ont une forme globalement aplanie.

Les deux ailes 36a et 36b de la couple d'ailes 36a, 36b de l'empreinte femelle 11 a sont légèrement divergentes à partir de l'âme 35, de manière que l'ouverture de l'empreinte femelle 11 b soit légèrement évasée. Ces ailes 36a et 36b sont reliées à l'âme 35 par deux parties arrondies 39 de rayon de courbure suffisamment important.

Les deux ailes 38a et 38b de la couple d'ailes 38a, 38b de l'empreinte mâle 11 b sont légèrement convergentes à partir de la seconde âme 7b qui constitue la base de la saillie 15. Ces ailes 38a et 38b sont reliées à l'âme 37 par deux parties arrondies 40 de rayon de courbure suffisamment important.

Les faces 13 et 14 ont des contours globalement analogues, comme il est décrit. Elles sont destinées à être disposées globalement dans la même direction. Elles sont écartées transversalement l'une de l'autre, par suite de la présence entre elles du tube T positivement pincé et fermé, à raison de la géométrie qu'elles présentent. Elles sont maintenues ainsi grâce à des moyens de verrouillage sur lesquels on reviendra.

Le dispositif 1 de pincement de fermeture pour le sectionnement et ses parties constitutives (sections 3 et parties 4 et 5), peuvent se trouver dans l'un des deux états suivants : état ouvert et état fermé.

A l'état ouvert (figure 1), les deux sections 3a et 3b sont écartées et dissociées l'une de l'autre. Les deux sections 3a et 3b peuvent alors être manipulées chacune séparément. Dans cet état ouvert, les deux sections 3a et 3b laissent un libre accès transversal aux empreintes 11a et 11b, chacune d'elle n'étant pas recouverte par la section à laquelle appartient l'autre empreinte.

A l'état ouvert, le tube T peut, dans toute région longitudinale souhaitée pour être une région de pincement RP, être introduit dans, ou si nécessaire enlevé de, l'empreinte femelle 11a formant berceau, sans que sa forme ne soit affectée, en particulier qu'il soit pincé ou aplati, la circulation, le passage de sectionnement, la communication du fluide dans le tube T n'étant pas bloqué, empêché, ou entravé. Le berceau de l'empreinte femelle 11a est bien entendu adapté à la forme et à la dimension transversale du tube T.

Ainsi, l'état ouvert permet la phase de mise en place du tube T dans le dispositif 1 de pincement de fermeture pour le sectionnement, éventuellement son enlèvement (figure 1).

A l'état fermé (figures 3, 3A), les deux sections 3a et 3b sont proches l'une de l'autre et emboîtées, les deux rives 8a de la première section mâle 3a venant se loger avec contact de maintien, dans les deux rives 8b de la seconde section femelle 3b.

A l'état fermé, les deux faces extérieures 9 des premières rives 8a sont en contact et en appui sur les deux faces intérieures 10 des secondes rives 8b, et, le cas échéant - comme indiqué précédemment -, les deux petites faces 16 sont en contact et en appui sur les deux chants libres 17 des secondes rives 8b.

A l'état fermé, le dispositif 1 de pincement de fermeture pour le sectionnement présente une forme générale extérieure qui est sensiblement celle d'un parallélépipède à section transversale au moins sensiblement carrée, allongé le long de l'axe 6, et dont les quatre arêtes 18 s'étendant axialement, sont arrondies. Ce parallélépipède comporte deux extrémités libres ou faces transversales d'extrémité et distales 19 (figure 4).

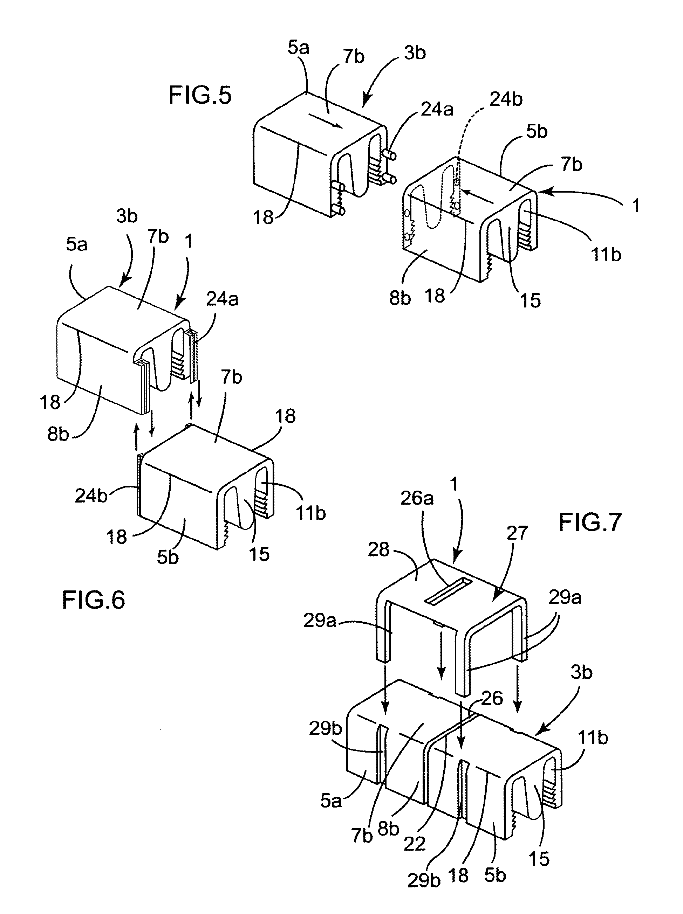

Chacune des parties 4a, 4b, 5a, 5b est terminée du côté extérieur par une extrémité libre ou face transversale d'extrémité distale 19 et, du côté intérieur, par une face transversale d'extrémité et proximale, à savoir, respectivement, 20a, 20b, 21a, 21 b (figures 4 et 5). Ces faces transversale d'extrémité et proximales sont substantiellement planes.

A l'état fermé, les deux parties 4a et 4b, 5a et 5b de chacune des deux sections 3a et 3b ont leurs deux faces transversales d'extrémité et proximales 20a et 20b, 21a et 21b situées en regard l'une de l'autre et à proximité immédiate l'une de l'autre mais néanmoins écartées l'une de l'autre, le long de l'axe longitudinal 6, de manière à ménager un passage transversal médian de sectionnement 22. Les deux faces transversales d'extrémité et proximales 20a et 21 a des parties 4a et 5a, comme les deux faces transversales d'extrémité et proximales 20b et 21 b des parties 4b et 5b sont coplanaires ou sensiblement coplanaires.

A l'état fermé, les deux empreintes 11 a et 11 b n'ont plus d'accès transversal libre comme dans l'état ouvert, mais elles ménagent entre elles un passage de pincement positif de fermeture 23, d'axe 6, formant une sorte de canal creux, limité par les deux faces 13 et 14 des empreintes 11a et 11b, et la partie des petites faces 16 attenantes à la saillie 15.

Dans cette réalisation, le passage de pincement positif de fermeture 23 est en deux tronçons de pincement positif de fermeture 23a placés dans la ligne de prolongement l'un de l'autre. Ces deux tronçons 23a débouchent tout d'abord des deux faces transversales d'extrémité et distales 19, vers l'extérieur du dispositif 1 de pincement de fermeture pour le sectionnement. Ces deux tronçons 23a débouchent ensuite des deux couples de faces transversales d'extrémité et proximales 20a, 21 a et 20b, 21 b.

Entre les deux couples de faces transversales d'extrémité et proximales 20a, 21 a et 20b, 21 b, le passage de pincement positif de fermeture 23 est absent pour ne pas exister, les couples de parties 4a, 5a et 4b, 5b étant écartés l'une de l'autre en direction axiale, sur une faible distance, comme indiqué précédemment.

Les deux tronçons de pincement positif de fermeture 23a sont donc placés dans le prolongement l'un de l'autre, mais avec un petit écartement entre eux 23b.

En se référant à la figure 2, on appelle par convention « zone de pincement positif de fermeture » ZPF du tube T, le tronçon longitudinal du tube T se trouvant au droit des parties 4a et 5a, 4b et 5b du dispositif 1 de pincement de fermeture pour le sectionnement, dans les deux tronçons de pincement positif de fermeture 23a du passage 23. On appelle « zone d'aplatissement » ZA du tube T, le tronçon longitudinal du tube T se trouvant entre les parties 4a et 5a, 4b et 5b du dispositif 1 de pincement de fermeture pour le sectionnement, dans l'écartement 23b entre les deux tronçons 23a du passage 23. La région de pincement RP du tube T déjà mentionnée est constituée par le tronçon longitudinal du tube T comprenant les deux zones de pincement positif de fermeture ZPF et la zone d'aplatissement ZA placé entre elles.

Dans une réalisation, les parties 4a et 4b d'une part, 5a et 5b d'autre part sont analogues, de sorte que le dispositif 1 de pincement de fermeture pour le sectionnement assure une fonction de fermeture du tube T sur lui-même dans les deux zones de pincement positif de fermeture ZPF, s'étendant sur une certaine longueur axiale, séparées l'une de l'autre par la zone d'aplatissement ZA. Dans cette zone, le tube T est maintenu, sans qu'une fermeture totale soit positivement assurée. Ce maintien du tube T facilite son sectionnement.

Dans une autre réalisation, on peut prévoir que le dispositif 1 de pincement de fermeture pour le sectionnement assure une fonction de fermeture du tube T sur lui-même dans une seule zone de pincement positif de fermeture ZPF. Ce qui, dans la réalisation précédente était l'autre zone de pincement positif de fermeture ZPF peut n'assurer que le maintien positif, sans fermeture totale, la zone d'aplatissement ZA jouant alors le même rôle que dans la réalisation précédente. Dans ce cas, il n'est pas indispensable que les parties creuses assurant ce simple maintien positif comportent des empreintes telles que celles précédemment décrites.

Le passage de pincement 23 a, en section droite transversale, une forme générale de U, par suite de la forme correspondante des empreintes 11a, 11b. Ce passage 23 comporte par conséquent une âme 41 et deux ailes 42a et 42b, correspondant, respectivement, aux âmes 35 et 37 et aux ailes 36a, 38a et 36b, 38b.

Le tube T disposé dans le passage de pincement 23 du dispositif 1 de pincement de fermeture pour le sectionnement à l'état fermé, contraint par le passage 23 profilé, est conformé en étant, d'une part aplati sur lui-même et ainsi fermé, d'autre part conformé en U, avec une âme Ta et deux ailes Tb, terminées par deux plis d'extrémité Tp.

La largeur (dans un plan transversal) de l'ouverture que forme le passage de pincement 23-âme 41 et ailes 42a et 42b - est définie par l'écartement transversal entre la face intérieure 13 et la face 14, à savoir l'écartement transversal entre les âmes 35 et 37, d'une part et les ailes en regard des couples d'ailes 36a et 38a, 36b et 38b.

A l'état fermé, les deux ailes 36a et 38a, 36b et 38b de chaque couple d'ailes en regard de l'empreinte mâle 11 a et de l'empreinte femelle 11 b vont en se rapprochant l'une de l'autre vers leurs extrémités 43 opposées aux âmes 35 et 37 des empreintes, au moins dans la zone de ces extrémités 43. Les sections 3a et 3b sont agencées en conséquence.

Dans la réalisation représentée, les deux ailes 36a et 38a, 36b et 38b vont en se rapprochant l'une de l'autre de façon plus ou moins continue depuis les âmes 35 et 37 jusqu'aux extrémités 43 opposées.

Par suite, chacune des deux ailes 42a et 42b du passage 23 a, entre les faces 36a, 38a et 36b, 38b des deux empreintes, une ouverture dont la largeur va en diminuant vers son extrémité opposée à l'âme 41 du passage 23, et notamment va en diminuant de façon plus ou moins continue à partir de l'âme 41.

La plus grande largeur du passage 23 est légèrement plus petite que le double de l'épaisseur de la paroi du tube T, en fonction de la compressibilité du tube T, de manière que le tube T une fois placé dans le dispositif 1 de pincement de fermeture pour le sectionnement à l'état fermé soit comprimé sur lui-même et que le dispositif 1 de pincement de fermeture pour le sectionnement assure une fonction de fermeture du tube T sur lui-même.

Par suite du fait que la largeur du passage 23 va en diminuant, vers son extrémité opposée à l'âme 41, le tube T est davantage comprimé dans la zone de ses deux plis Tp. Cette disposition permet d'éviter que ne subsiste dans la zone des plis une petite ouverture du tube sur lui-même, une telle ouverture ayant pour effet de permettre à une ou plusieurs gouttes du fluide du tube T de s'écouler de lui lors du pincement de fermeture et le sectionnement du tube T.

Ainsi, le dispositif 1 de pincement de fermeture pour le sectionnement de pincement remplit une fonction de fermeture du tube T sur lui-même dans la région de pincement empêchant tout passage du fluide dans le tube T.

Une fois le tube T, dans la phase de mise en place, positionné dans l'empreinte femelle 11a, alors que le dispositif 1 de pincement de fermeture pour le sectionnement est à l'état ouvert, le dispositif 1 de pincement de fermeture pour le sectionnement est amené à l'état fermé, le tube T est emprisonné dans le passage 23 dans la phase de pincement.

Dans la phase de pincement, le tube T est pincé positivement et fermé dans ses deux zones de pincement positif de fermeture ZPF par les faces 13 et 14 et la coopération des empreintes 11 a et 11 b. Entre les deux zones de pincement positif de fermeture ZPF, le tube T n'est pas positivement pincé et fermé, mais il est aplati plus ou moins fortement avec un certain degré, ce qui justifie que ce tronçon de tube T soit qualifié de zone d'aplatissement ZA. Par suite du pincement positif de fermeture, le tube T est fermé sur lui-même dans la région de pincement RP, ce qui empêche la circulation, le passage, la communication du fluide de part et d'autre de cette région.

Avec le au moins un organe de coupe 2, le tube T est, dans une phase de sectionnement ultérieure à la phase de pincement, sectionné transversalement dans le passage de sectionnement 22, dans la zone d'aplatissement ZA et donc dans la région de pincement RP. Le sectionnement intervenant après la fermeture, il n'est en aucune manière perturbé par le fluide se trouvant dans le tube T. La fermeture étant totale, le risque que s'écoulent une ou plusieurs gouttes du fluide du tube T est évité.

Les formes et dimensions du dispositif 1 de pincement de fermeture pour le sectionnement sont adaptées à la nature, à la forme et aux dimensions du tube T, de manière que le pincement de fermeture souhaité en ce qui concerne le tube T soit correctement réalisé.

D'autre part, la forme et les dimensions de l'organe de coupe 2 sont choisies de manière que l'organe de coupe 2 puisse via l'accès 26 passer dans le passage transversal de sectionnement 22 du dispositif 1 de pincement de fermeture pour le sectionnement et ainsi sectionner le tube pincé et fermé par ce dispositif 1.

Dans la réalisation de la figure 1, l'empreinte femelle 11 a et l'empreinte mâle 11 b ont une même forme, une même disposition et de mêmes dimensions, constantes et uniformes, d'une extrémité libre et distale 19 à l'autre extrémité libre et distale 19, via les extrémités proximales.

Afin de pouvoir être placées et maintenues dans le prolongement l'une de l'autre (figure 1), les deux parties 4a et 4b ou 5a et 5b d'une même section 3a ou 3b sont structurellement et fonctionnellement associées entre elles, de façon amovible, par des moyens d'association.

Plus spécialement, les deux parties 4a et 4b ou 5a et 5b sont associés par leurs faces d'extrémité et proximales, respectivement 20a et 20b d'une part, 21 a et 21b d'autre part (écartées le long de l'axe 6 pour former le passage de sectionnement 22), grâce à des moyens d'association 24a et 24b appartenant aux parties 4a et 4b d'une part, 5a et 5b d'autre part.

Dans la réalisation des figures 1 et 5, les moyens d'association 24 comprennent plusieurs (ici quatre mais pouvant être d'un nombre différent) saillies ou ergots formant tétons 24a ménagés sur la face d'extrémité et proximale 20a, 20b, 21a, 21b de l'une des deux parties 4a, 4b, 5a, 5b, aptes à coopérer de façon amovible avec un nombre égal de trous borgnes ou creux formant cavités 24b, complémentaires, ménagés sur la face d'extrémité et proximale 20b, 20a, 21b, 21a de l'autre des deux parties 4b, 4a, 5b, 5a.

Ces saillies 24a et ces trous 24b s'étendent parallèlement à l'axe 6, et donc à la direction d'extension du tube T lorsqu'il est positionné dans le dispositif 1 de pincement de fermeture pour le sectionnement. Leur coopération est réalisée par un coulissement axial parallèlement à l'axe 6. Leurs dimensions sont telles que les saillies 24a viennent se loger avec le jeu minimal dans les trous 24b.

Pour que le maintien des parties 4a et 4b d'une part, 5a et 5b d'autre part soit assuré de façon efficace, les saillies 24a et les trous 24b sont disposées sur les paires de rives 8a d'une part, 8b d'autre part, de part et d'autre des empreintes 11a et 11 b et du passage de sectionnement 22.

Afin de ne pas obstruer l'accès et l'ouverture du passage de sectionnement 22, il est prévu qu'aucune saillie et aucun trou tel que 24a, 24b ne se trouve au droit des âmes 7a et 7b.

Selon d'autres réalisations, le nombre de saillies 24a et de trous 24b est différent, ou il est prévu sur une même face d'extrémité et proximale donnée 20a, 20b, 21 a, 21 b, non pas un ou plusieurs saillies 24a ou trous 24b, mais une combinaison de saillies 24a et de trous 24b.

Le passage transversal de sectionnement 22 est réalisé par la présence de moyens d'écartement prévus à cet effet. Ces moyens d'écartement sont constitués par des butées ménagés soit sur les faces d'extrémité et proximales 20a, 20b, 21 a, 21 b soit sur les moyens d'association 24a, 24b, par exemple, les saillies 24a sont bloquées en fin de course dans les trous 24b.

Dans la réalisation du dispositif 1 de pincement de fermeture pour le sectionnement représenté sur la figure 1, les deux sections 3a et 3b comprennent des moyens 25a et 25b de verrouillage réciproque des deux sections 3a et 3b et des parties 4a et 5a à l'état fermé. Ces moyens de verrouillage 25a et 25b sont irréversibles, les deux sections 3a et 3b une fois amenées à l'état fermé étant maintenues dans cet état par les moyens de verrouillage 25a et 25b, sans pouvoir être dissociées.

Ainsi, une fois que le tube T logé dans le passage 23 a été pincé et fermé, le dispositif 1 de pincement de fermeture pour le sectionnement ayant été amené à l'état fermé, le tube T est maintenu ainsi pincé et fermé et il reste dans cet état, le dispositif 1 de pincement de fermeture pour le sectionnement restant associé au tube T par suite de l'irréversibilité des moyens de verrouillage 25a et 25b.

Dans la réalisation de l'ensemble de pincement de fermeture et de sectionnement représenté sur la figure 1, dans laquelle le pincement positif de fermeture intervient dans deux zones de pincement positif de fermeture ZPF, les deux sections 3a et 3b comprennent des moyens 25a et 25b de verrouillage réciproque des deux sections 3a et 3b et des deux couples de parties 4a, 5a et 4b, 5b à l'état fermé.

Dans une variante de cette réalisation, l'irréversibilité des moyens de verrouillage 25a et 25b est prévue pour l'une seulement des deux couples de parties 4a, 5a et 4b, 5b. Cette variante est utile lorsque l'on souhaite qu'une extrémité de sectionnement soit fermée sans nécessité que l'autre extrémité de sectionnement le soit, les parties correspondantes des sections 3a et 3b pouvant alors être enlevées.

Dans une autre réalisation d'un ensemble de pincement de fermeture et de sectionnement où le dispositif 1 de pincement de fermeture pour le sectionnement assure une fonction de fermeture du tube T sur lui-même dans une seule zone de pincement positif de fermeture ZPF, l'autre zone n'assurant qu'un maintien sans fermeture totale, on peut également prévoir que l'irréversibilité des moyens de verrouillage 25a et 25b n'est prévue que pour les parties des sections 3a et 3b assurant le pincement positif de fermeture, mais n'est pas prévue pour les parties n'assurant qu'un maintien sans fermeture totale.

Dans la réalisation des figures 1 et 5, les moyens de verrouillage réciproque 25a et 25b sont deux ensembles comprenant une série de dents d'accrochage irréversible profilées (ou des crans), orientées selon deux directions opposées, s'étendant axialement, ménagées respectivement sur les deux faces extérieures 9 des premières rives 8a et les deux faces intérieures 10 des secondes rives 8b dans leur zone d'emboîtement.

A la place des dents, on peut prévoir des crans ou analogue.

Dans la réalisation des figures 1, 3 et 3A, les moyens de verrouillage réciproque 25a et 25b sont amenés à coopérer l'un avec l'autre consécutivement à un mouvement de coulissement de rapprochement des deux sections 3a et 3b selon une direction transversale, conduisant à l'emboîtement à force des deux sections 3a et 3b, les dents 25a et 25b venant en prise mutuellement de façon irréversible.

Le passage de sectionnement 22 débouche sur la face extérieure du dispositif 1 de pincement de fermeture pour le sectionnement par au moins un accès, en l'espèce une fente d'accès 26, apte à permettre l'introduction dans le passage de sectionnement 22, et l'enlèvement depuis le passage de sectionnement 22, de l'organe de coupe 2, par un mouvement de coulissement transversal ou d'un organe de commande du mouvement de l'organe de coupe 2. D'autre part, le passage de pincement 23 débouche dans le passage de sectionnement 22.

Dans la réalisation des figures 1 et 5, le passage de sectionnement 22 est traversant de la fente 26 à une fente analogue diamétralement opposée située également à la périphérie du dispositif 1 de pincement de fermeture pour le sectionnement. Dans une autre réalisation non représentée, le passage de sectionnement 22 est non traversant ou borgne, ne débouchant pas à la périphérie diamétralement opposée du dispositif 1 de pincement de fermeture pour le sectionnement, auquel cas il n'y a qu'une seule fente 26.

Lorsqu'il est prévu deux fentes diamétralement opposées, on peut, selon les réalisations prévoir un seul organe de coupe 2 (ou organe de commande du mouvement de l'organe de coupe 2) ou deux organes de coupe 2 opposés (ou organes de commande), chacun étant introduit par une fente dédiée.

Le passage de sectionnement 22 débouche également dans, et interfère avec, l'espace ou écartement 23a situé entre les deux tronçons de pincement positif de fermeture 23a.

L'accès 26, disposé transversalement, comme le passage de sectionnement 22, comporte un grand côté ou longueur et un petit côté ou largeur. Le grand côté s'étend transversalement au droit de l'ouverture du berceau de la face 13 de la première empreinte 11a, et a une longueur au moins légèrement plus grande que cette ouverture. Avec cette disposition constructive, on est assuré que l'organe de coupe 2 vient bien interférer avec la totalité d'une section transversale du tube T maintenu pincé et fermé dans les deux tronçons de pincement positif de fermeture 23a et traversant l'espace ou écartement 23b situé entre eux. Et, ainsi, l'organe de coupe 2 peut sectionner transversalement le tube T dans l'espace ou écartement 23b.

A cet effet, les moyens d'association 24a, 24b des sections 3 sont espacés de la face 13, de manière à permettre le passage de l'organe de coupe 2 entre les moyens d'association 24a, 24b, sans les couper ou même les entamer.

Le petit côté de l'accès 26 est disposé axialement. Sa longueur, faible, est adaptée à l'épaisseur de l'organe de coupe 2 (ou de l'organe de commande du mouvement de l'organe de coupe 2), de manière à permettre le passage et le guidage de l'organe de coupe 2 (ou de l'organe de commande) dans le passage de sectionnement 22, sans que l'organe de coupe 2 ne coupe ou même n'entame le dispositif 1 de pincement de fermeture pour le sectionnement lui-même.

Le passage de sectionnement 22 et de l'accès 26 sont dimensionnés en fonction des dimensions du dispositif 1 de pincement de fermeture pour le sectionnement, de la nature de la forme et des dimensions du tube T, ainsi que des dimensions de l'organe de coupe 2 ou de l'organe de commande de l'organe de l'organe de coupe 2, de manière à assurer un sectionnement efficace.

Bien entendu, il est prévu les jeux nécessaires au mouvement de l'organe de coupe.

Le dispositif 1 de pincement de fermeture pour le sectionnement qui vient d'être décrit est mis en oeuvre comme il est maintenant décrit, dans le but de pincer et de fermer un tube T et, en combinaison avec le au moins un organe de coupe 2, dans le cadre d'un ensemble de pincement de fermeture et de sectionnement, dans le but de couper transversalement le tube T.

On dispose d'un dispositif 1 de pincement de fermeture pour le sectionnement qui est, ou est amené, à l'état ouvert et dont chaque section 3a et 3b comporte ses deux parties 4a et 4b, et 5a et 5b associées grâce aux moyens d'association 24a et 24b.

On dispose du tube T, qui, dans cette situation est continu et normalement conformé pour laisser passer le fluide.

On positionne le tube T, dans la région de pincement RP souhaitée de celui-ci, dans l'empreinte femelle 11 a de la première section mâle 3a (phase de mise en place). Par exemple, la première section mâle 3a est disposée pour que sa face 13 soit tournée frontalement vers l'opérateur ou soit tournée pour être à la vue de l'opérateur. Dans la réalisation de la figure 1, la première section mâle 3a est disposée sensiblement avec l'axe 6 horizontal, sa face 13 tournée vers le haut.

On amène la seconde section femelle 3b en regard de la première section mâle 3a, les faces 13 et 14 étant tournées l'une vers l'autre. Dans la réalisation de la figure 1, la seconde section femelle 3b est disposée au dessus de la première section mâle 3a, sensiblement avec l'axe 6 horizontal, sa face 14 tournée vers le bas à l'aplomb de la face 13.

Par un mouvement relatif de rapprochement des première et seconde sections 3a et 3b, et des parties 4a et 4b d'une part, 5a et 5b d'autre part, en l'espèce par un mouvement relatif de coulissement transversal, on fait passer le dispositif 1 de pincement de fermeture pour le sectionnement (ses sections 3 et parties 4 et 5) de l'état ouvert antérieur à l'état fermé ultérieur. Dans la réalisation de la figure 1, ce mouvement relatif de coulissement transversal est un mouvement vertical.

Ce mouvement de fermeture est poursuivi jusqu'en fin de course, alors que le tube T est positivement pincé jusqu'à être fermé dans ses deux zones de pincement positif de fermeture ZPF par les faces 13 et 14 qui le déforment et le contraignent ainsi, étant donné la relation géométrique existant entre le tube T et les deux tronçons de pincement positif de fermeture 23a du passage 23 (phase de pincement).

Ce mouvement de fermeture est rendu aisé par suite des moyens de verrouillage 25a, 25b, irréversible, c'est-à-dire anti-retour.

En amenant le dispositif 1 de pincement de fermeture pour le sectionnement à l'état fermé, on fait simultanément coopérer les moyens de verrouillage réciproque 25a et 25b, pour maintenir fermées les deux parties 4a et 5a d'une part, 4b et 5b d'autre part, de chaque paire de parties des première et seconde sections 3a et 3b, dans la position où le tube T est pincé et fermé.

On maintient le tube T ainsi pincé et fermé entre les deux sections 3a et 3b du dispositif 1 de pincement de fermeture pour le sectionnement.

Comme indiqué, on souhaite également sectionner le tube T dans la région de pincement RP afin d'obtenir un tube T sectionné en deux longueurs, chacune d'elle pincée et fermée à sa partie extrême sectionnée ou, comme indiqué plus haut, l'une seulement étant pincée et fermée, l'autre étant seulement maintenue. On dispose à cet effet du au moins un organe de coupe 2.

Alors que le dispositif 1 de pincement de fermeture pour le sectionnement est à l'état fermé autour du tube T, dans une phase de sectionnement, on coupe le tube T après que l'on a réalisé son pincement et sa fermeture, dans la zone d'aplatissement ZA dans laquelle le tube n'est pas positivement pincé, mais néanmoins aplati. Selon les réalisations, on sectionne le tube T immédiatement après ou un certain temps après que l'on a réalisé son pincement positif de fermeture.

A cet effet, on amène l'organe de coupe 2 en regard de l'accès 26 du dispositif 1 de pincement de fermeture pour le sectionnement.

Puis, par un mouvement relatif de rapprochement de l'organe de coupe 2 et du dispositif 1 de pincement de fermeture pour le sectionnement, en l'espèce un mouvement de coulissement transversal de l'organe de coupe 2 dans son propre plan, on introduit l'organe de coupe 2 par son extrémité dans l'accès 26 et on poursuit le mouvement jusqu'à ce que l'organe de coupe 2 pénètre davantage dans le passage de sectionnement 22, puis dans l'espace ou écartement 23b où il interfère avec le tube T dans sa zone d'aplatissement ZA entre les paires de faces proximales 20a, 21a et 20b, 21b. C'est ainsi que l'on coupe ou sectionne le tube T transversalement.

Dans ce mouvement, l'organe de coupe 2 est, le cas échéant, guidé par les paires de faces proximales 20a, 21 a et 20b, 21 b, et le cas échéant également, par les organes d'association 24a, 24b.

Ainsi, on sectionne le tube T, sans que l'organe de coupe 2 ne sectionne, ne pénètre dans, ou n'interfère frontalement avec le dispositif 1 de pincement de fermeture pour le sectionnement. Par suite, on évite l'apparition de copeaux ou déchets gênants provenant du dispositif 1 de pincement de fermeture pour le sectionnement.

Selon les réalisations, le mouvement de coulissement de l'organe de coupe 2 est poursuivi de part en part du dispositif 1 de pincement de fermeture pour le sectionnement si le passage de sectionnement 22 est traversant ou est arrêté à la position adéquate si le passage de sectionnement 22 est non traversant, borgne. Selon les réalisations, la coupe est faite au moyen d'un seul organe de coupe 2 ou de deux organes de coupe 2 opposés.

Selon les réalisations, on fait passer l'organe de coupe 2 dans le passage de sectionnement 22 sans contact avec le dispositif 1 de pincement de fermeture pour le sectionnement (figure 2) ou au contraire avec un contact surfacique glissant avec le dispositif 1 de pincement de fermeture pour le sectionnement ((figure 9).

Une fois le tube T sectionné dans sa zone d'aplatissement ZA, l'organe de coupe 2 peut être enlevé, soit parce qu'il a traversé de part en part du dispositif 1 de pincement de fermeture pour le sectionnement si le passage de sectionnement 22 est traversant, soit par un mouvement de coulissement dans le sens opposé au sens d'introduction, si le passage de sectionnement 22 est non traversant, borgne.

Dans cette situation, il est alors possible de dissocier les parties 4a et 4b d'une part et les parties 5a et 5b d'autre part, en dissociant les uns des autres les moyens d'association 24a et 24 b par un mouvement de coulissement axial d'écartement (phase de dissociation). Toutefois, on laisse les parties 4a et 5a d'une part, 4b et 5b d'autre part fixées aux parties extrêmes sectionnées du tube T en les pinçant et en les fermant.

On obtient alors un tube T sectionné en deux tronçons ou longueurs, chacun pincé et fermé à sa partie extrême sectionnée, à laquelle est fixée à cette fin une couple de parties, respectivement 4a et 5a d'une part, 4b et 5b d'autre part.

Ou, comme indiqué, on obtient un tube T sectionné en deux tronçons ou longueurs, qui d'un seul côté est pincé et fermé.

Selon une variante de réalisation, le passage de pincement 23 est d'ouverture réglable pour s'adapter à des tubes T de différentes géométries, notamment tailles.

A cet effet, il peut être prévu dans une réalisation de disposer de différents dispositifs 1 correspondant à des géométries, notamment tailles, différentes, ce qui permet de couvrir un large spectre de géométries de tubes T.

Dans une autre réalisation (figure 3), il est prévu que le mouvement relatif de rapprochement et de fermeture des première et seconde sections 3a et 3b est plus ou moins grand, de sorte que le passage de pincement 23 est, respectivement, plus ou moins petit. Cela est rendu possible par suite de l'existence de moyens de verrouillage réciproque 25a et 25b, comprenant une série de dents d'accrochage profilées étagées entre l'âme 7a, 7b de la section 3a, 3b et le bord libre de chant de la rive 8a, 8b attenante, ce qui permet par un anti retour, différentes positions relatives de verrouillage des sections 3a et 3b.

Dans une réalisation (figure 4), il est prévu que l'une ou/et l'autre des deux empreintes 11a et 11b est légèrement inclinée le long de l'axe 6, vers le bord libre de chant de la rive 8a, 8b et vers l'extrémité proximale 20a, 20b, 21 a, 21 b.

Cette disposition constructive permet d'exercer un pincement du tube T plus important vers cette extrémité proximale 20a, 20b, 21a, 21b, ce qui est de nature à expulser le fluide se trouvant dans le tube T vers l'autre extrémité, distale 19.

Selon une réalisation (figure 6), les moyens d'association 24a et 24b comprennent une saillie 24a et une rainure 24b, ménagées sur les faces d'extrémité et proximales 20a, 20b, 21a, 21b, s'étendent non parallèlement à l'axe 6 comme dans la réalisation des figures 1 et 5, mais s'étendant transversalement. Dans ce cas, le mouvement d'association ou de dissociation est un mouvement de coulissement transversal parallèlement à la direction de la saillie 24a et de la rainure 24b, et non un mouvement axial.

Selon une autre réalisation (figure 7), les moyens d'association 27 diffèrent des moyens d'association à saillie 24a et trou ou rainure 24b, comme décrit précédemment, et comprennent une pièce amovible de liaison en forme de clip 27, apte à associer de façon amovible les deux parties 4a et 4b ou 5a et 5b de façon non pas directe mais indirecte.

Ce clip 27 comprend une âme plate 28, apte à venir, lorsque le clip est monté en situation d'association, contre la face extérieure du dispositif 1 de pincement de fermeture pour le sectionnement. Cette âme 28 est percée d'une fente 26a apte à venir à l'aplomb de l'accès 26 et à se superposer à lui.

De l'âme 28 sont attenants quatre (ou un plus grand nombre) saillies en forme de tige 29a, profilées, parallèles entre elles et orthogonales à l'âme 28. Ces saillies 29a sont aptes à coopérer avec un nombre égal de rainures 29b, transversales, ménagées sur la face extérieure du dispositif 1 de pincement de fermeture pour le sectionnement, sur chaque partie 4a, 4b, 5a, 5b.

Dans les différentes réalisations précédemment décrites, les deux parties 4a et 5a d'une part, 4b et 5b d'autre part, des deux sections 3a et 3b, sont des pièces distinctes et le mouvement relatif de fermeture est un mouvement de coulissement transversal en translation.

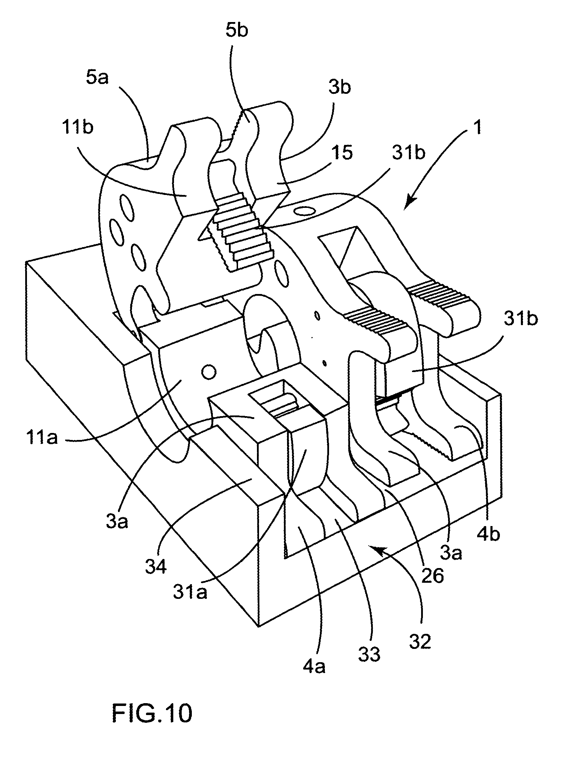

Dans une autre réalisation (figures 9 et 10), les deux parties 4a et 5a d'une part, 4b et 5b d'autre part, des deux sections 3a et 3b, sont des pièces non pas distinctes mais associées structurellement l'une à l'autre à pivotement autour d'un axe de pivotement 30 parallèle à l'axe 6 et le mouvement relatif de fermeture n'est pas un mouvement de coulissement transversal en translation mais un mouvement de pivotement autour de l'axe de pivotement 30. L'axe de pivotement 30 est déporté très largement par rapport à l'axe 6, sur un côté longitudinal du dispositif 1 de pincement de fermeture pour le sectionnement, de sorte que, le rayon de pivotement étant important, la fin du mouvement de pivotement d'emboîtement diffère peu d'un coulissement transversal, comme précédemment.

Du côté longitudinal opposé à l'axe 6, il est prévu des moyens de verrouillage 25a et 25b du type comportant, pour l'un, une pièce femelle 31a, rapportée sur l'une des parties 4a, 5a ou 4b, 5b, et pourvue de dents 25a et, pour l'autre, d'une pièce mâle 31 b, rapportée sur l'autre des parties 5a, 4a ou 5b, 4b, et pourvue de dents 25b.

Les dents 25a, 25 b, comme précédemment, sont des dents d'accrochage irréversible profilées, orientées selon deux directions opposées, s'étendant axialement.

Dans la réalisation des figures 9 et 10, comme dans la réalisation de la figure 7, les moyens d'association 32 comprennent, à l'instar de la pièce 27, une pièce amovible de liaison en forme de berceau 32, apte à associer de façon amovible les deux parties 4a et 4b ou 5a et 5b de façon non pas directe mais indirecte.

Cette pièce 32 comprend un berceau 33, apte à venir, lorsque la pièce 32 est montée en situation d'association, contre la face extérieure du dispositif 1 de pincement de fermeture pour le sectionnement, sur un côté de celui-ci, de manière à laisser libre l'autre côté ou est accessible le passage de sectionnement 22.

Le berceau 33 comporte deux rives 34 se faisant face, aptes à venir enserrer les parties 4a et 4b ou 5a et 5b par leurs faces extrêmes distales 19.

Dans les réalisations précédemment décrites, le dispositif 1 de pincement de fermeture pour le sectionnement n'inclut pas structurellement le au moins un organe de coupe 2, celui-ci étant toutefois associé fonctionnellement.

Selon d'autres réalisations, le dispositif 1 de pincement de fermeture pour le sectionnement inclut structurellement et fonctionnellement au moins un organe de coupe 2. Par exemple, dans le cas de la figure 8, l'organe de coupe 2 forme une sorte de guillotine ayant en élévation une forme générale de U, est montée à coulissement dans son propre plan sur la face d'extrémité proximale 20a ou 20b de l'une des parties 4a, 4b pourvue de moyen de guidage appropriés. Dans cette réalisation, il est prévu un seul organe de coupe 2 ou deux organes de coupe sur les deux faces d'extrémité 20a et 20b. Dans cette réalisation, l'accès 26 sert au passage d'un organe de commande du mouvement de l'organe de coupe 2, tel qu'une sorte de lame.

## Revendications

1. Procédé pour sectionner et fermer un tube souple, dans lequel :
- on dispose du tube à sectionner et à fermer,
- on dispose d'un ensemble de pincement de fermeture et de sectionnement comprenant un dispositif (1) de pincement de fermeture pour le sectionnement et au moins un organe de coupe (2), le dispositif (1) de pincement de fermeture pour le sectionnement comprenant deux sections (3a, 3b), chacune ayant deux parties creuses (4a, 4b et 5a, 5b), une couple au moins (4a et 5a ou 4b et 5b) étant à empreintes (11a, 11b), ces parties (4a, 4b et 5a, 5b) pouvant être placées dans le prolongement l'une de l'autre par des moyens d'association (24a, 24b, 27, 32) tout en ménageant entre elles un passage transversal de sectionnement (22) ayant un accès (26) apte au passage de l'organe de coupe (2) ou d'un organe de commande de l'organe de coupe (2), le dispositif (1) de pincement de fermeture pour le sectionnement pouvant se trouver soit à l'état ouvert où les deux sections (3a, 3b) sont écartées l'une de l'autre soit à l'état fermé où elles sont rapprochées et fermées l'une sur l'autre et maintenues fermées par des moyens de verrouillage (25a, 25b) et où les faces (13, 14) des empreintes (11a, 11b) forment un passage (23) de pincement et de fermeture du tube,
- on dispose du dispositif (1) de pincement de fermeture pour le sectionnement à l'état ouvert,
- on positionne une région de pincement du tube dans l'empreinte (11a) d'une section (3a) et on amène l'autre section (3b) en regard de la précédente et le dispositif (1) de pincement de fermeture pour le sectionnement à l'état fermé, avec coopération des moyens de verrouillage (25a, 25b), jusqu'à ce que le tube soit positivement pincé et fermé dans au moins une zone de pincement positif de fermeture par les faces (13, 14), et on maintient le tube ainsi pincé et fermé entre les sections (3a, 3b),
- on sectionne le tube entre les parties (4a et 5a, 4b et 5b) à l'état fermé, à l'aide du au moins un organe de coupe (2),
- on dissocie les moyens d'association (24a, 24b) et donc les parties (4a et 4b, 5a et 5b), dont la au moins une couple de parties (4a et 5a ou 4b et 5b) à empreintes (11 a, 11b) reste fixée à la partie extrême sectionnée du tube en la pinçant et en la fermant,
**caractérisé par le fait que** :
- on dispose d'un dispositif (1) de pincement de fermeture pour le sectionnement dont l'une des empreintes (11a) est une empreinte femelle, l'autre des empreintes est une empreinte mâle (11b), les deux empreintes (11a, 11b), à l'état fermé du dispositif (1) de pincement de fermeture pour le sectionnement ménageant entre elles un passage de pincement positif de fermeture (23) limité par les deux faces (13, 14) des empreintes (11 a, 11 b), ayant, en section droite transversale, une forme générale de U,
- et, pour sectionner le tube, on fait passer le au moins un organe de coupe (2) dans le passage de sectionnement (22) sans que l'organe de coupe (2) ne sectionne, ne pénètre dans, ou n'interfère frontalement avec le dispositif (1) de pincement de fermeture pour le sectionnement.

2. Procédé selon la revendication 1, **caractérisé par le fait que** l'on sectionne le tube immédiatement après ou un certain temps après que l'on a réalisé son pincement positif de fermeture.

3. Procédé selon l'une quelconque des revendications 1 et 2, **caractérisé par le fait que** l'on fait passer l'organe de coupe (2) dans le passage de sectionnement (22) soit sans contact avec le dispositif (1) de pincement de fermeture pour le sectionnement, soit avec un contact surfacique glissant avec le dispositif (1) de pincement de fermeture pour le sectionnement.

4. Procédé selon l'une quelconque des revendications 1 à 3, **caractérisé par le fait que** l'on sectionne le tube au moyen d'un seul organe de coupe (2) ou de deux organes de coupe (2) opposés.

5. Procédé selon l'une quelconque des revendications 1 à 4, **caractérisé par le fait que** l'on sectionne le tube dans une zone d'aplatissement dans laquelle on ne pince pas positivement le tube.

6. Procédé selon l'une quelconque des revendications 1 à 5, **caractérisé par le fait que** l'on amène une section (3b) en regard d'une autre (3a) et le dispositif (1) de pincement de fermeture pour le sectionnement à l'état fermé, par un mouvement relatif de translation transversale ou de pivotement autour d'un axe (30) qui, par rapport à l'axe longitudinal (6) du dispositif (1) de pincement de fermeture pour le sectionnement est déporté sur un côté longitudinal du dispositif (1) de pincement de fermeture pour le sectionnement.

7. Procédé selon l'une quelconque des revendications 1 à 6, **caractérisé par le fait que** soit l'on maintient le tube positivement pincé et fermé dans une seule zone de pincement positif de fermeture et on maintient le tube dans l'autre zone, soit l'on maintient le tube positivement pincé et fermé dans deux zones de pincement positif de fermeture.

8. Procédé selon l'une quelconque des revendications 1 à 7, **caractérisé par le fait que** l'on dissocie les moyens d'association (24a, 24b, 27, 32) et donc les parties (4a et 4b, 5a et 5b), alors que soit l'une seulement des couples de parties (4a, 5a et 4b, 5b) à empreintes (11a, 11 b) reste fixée à la partie extrême sectionnée du tube en la pinçant et en la fermant, soit les deux couples de parties (4a, 5a et 4b, 5b) à empreintes (11 a, 11 b) restent fixées à la partie extrême sectionnée du tube en la pinçant et en la fermant.

9. Procédé selon l'une quelconque des revendications 1 à 8, **caractérisé par le fait que** l'on commence le pincement de fermeture du tube dans la zone des deux plis latéraux du tube.

10. Dispositif (1) de pincement de fermeture pour le sectionnement spécialement destiné, en combinaison avec un organe de coupe (2) à la mise en oeuvre du procédé selon l'une quelconque des revendications 1 à 9, **caractérisé en ce qu'**il comprend deux sections (3a, 3b), chacune ayant deux parties creuses (4a, 4b et 5a, 5b), une couple au moins (4a et 5a ou 4b et 5b) étant à empreintes (11a, 11b), ces parties (4a, 4b et 5a, 5b) pouvant être placées dans le prolongement l'une de l'autre de façon amovible par des moyens d'association (24a, 24b, 27, 32) tout en ménageant entre elles un passage transversal de sectionnement (22) ayant un accès (26) apte au passage de l'organe de coupe (2) ou d'un organe de commande de l'organe de coupe (2), le passage transversal de sectionnement (22) étant ménagé entre les deux couples de faces transversales d'extrémité et proximales (20a, 20b, 21 a 21b) des sections (3a, 3b) situées en regard l'une de l'autre et écartées à proximité l'une de l'autre le long de l'axe (6) du dispositif (1) de pincement de fermeture pour le sectionnement, lequel peut se trouver soit à l'état ouvert où les deux sections (3a, 3b) sont écartées l'une de l'autre soit à l'état fermé où elles sont rapprochées et fermées l'une sur l'autre et maintenues fermées par des moyens de verrouillage (25a, 25b) et où les faces (13, 14) des empreintes (11a, 11b) forment un passage (23) de pincement et de fermeture du tube comportant au moins un tronçon (23a) auquel est adjacent axialement un espace (23b) dans lequel débouche le passage (22) et où le tube est d'une part maintenu et d'autre part aplati avec un certain degré, l'une des empreintes (11 a) étant une empreinte femelle, l'autre des empreintes étant une empreinte mâle (11b), les deux empreintes (11a, 11b), à l'état fermé du dispositif (1) de pincement de fermeture pour le sectionnement ménageant entre elles un passage de pincement positif de fermeture (23) limité par les deux faces (13, 14) des empreintes (11 a, 11 b), ayant, en section droite transversale, une forme générale de U.

11. Dispositif (1) de pincement de fermeture pour le sectionnement selon la revendication 10, **caractérisé par le fait que** l'une des empreintes (11a) est une empreinte femelle, l'autre des empreintes est une empreinte mâle (11b), les deux empreintes (11 a, 11b), à l'état fermé du dispositif (1) de pincement de fermeture pour le sectionnement ménageant entre elles un passage de pincement positif de fermeture (23) limité par les deux faces (13, 14) des empreintes (11a, 11 b), ayant, en section droite transversale, une forme générale de U.

12. Dispositif (1) de pincement de fermeture pour le sectionnement selon l'une quelconque des revendications 10 et 11, **caractérisé par le fait que** la largeur de l'ouverture du passage de pincement (23), définie par l'écartement transversal entre les faces (13 et 14) de section droite transversale en forme générale de U, est légèrement plus petite que le double de l'épaisseur de la paroi du tube souple à pincer.

13. Dispositif (1) de pincement de fermeture pour le sectionnement selon l'une quelconque des revendications 10 à 12, **caractérisé par le fait que** :
- l'empreinte mâle (11b) a en section droite transversale une forme générale de U avec une âme et deux ailes, l'empreinte femelle (11a) a en section droite transversale une forme générale de U avec une âme et deux ailes et le passage (23) de pincement a en section droite transversale une forme générale de U avec une âme et deux ailes,
- les deux ailes en regard de chaque couple d'ailes en regard de l'empreinte mâle (11b) et de l'empreinte femelle (11a) vont en se rapprochant l'une de l'autre vers leurs extrémités opposées aux âmes des empreintes, au moins dans la zone de ces extrémités et chacune des deux ailes du passage (23) de pincement a, entre les faces des deux empreintes (11a et 11b) une ouverture dont la largeur va en diminuant vers son extrémité opposée à l'âme du passage (23) de pincement.

14. Dispositif (1) de pincement de fermeture pour le sectionnement selon l'une quelconque des revendications 10 à 13, **caractérisé en ce que** les deux parties creuses (4a, 4b et 5a, 5b) de chaque section (3a et 3b) sont soit analogues, le dispositif (1) de pincement de fermeture pour le sectionnement assurant une fonction de fermeture du tube sur lui-même dans deux zones de pincement positif de fermeture, soit différentes, le dispositif (1) de pincement de fermeture pour le sectionnement assurant une fonction de fermeture du tube sur lui-même dans une seule zone de pincement positif de fermeture et étant seulement maintenu dans l'autre zone.

15. Dispositif (1) de pincement de fermeture pour le sectionnement selon l'une quelconque des revendications 10 à 14, **caractérisé en ce que** les moyens de verrouillage (25a et 25b) sont soit irréversibles pour les deux couples de parties creuses (4a et 5a, 4b et 5b), soit irréversibles pour l'une des couples de parties creuses (4a et 5a ou 4b et 5b) et réversibles pour l'autre (4b et 5b ou 4a et 5a).

16. Dispositif (1) de pincement de fermeture pour le sectionnement selon l'une quelconque des revendications 10 à 15, **caractérisé par le fait que** le passage de pincement (23) est d'ouverture réglable, le mouvement relatif pour amener une section (3b) en regard d'une autre (3a) et le dispositif (1) de pincement de fermeture pour le sectionnement à l'état fermé étant plus ou moins grand, pour que le passage de pincement (23) soit respectivement, plus ou moins petit.

17. Dispositif (1) de pincement de fermeture pour le sectionnement selon l'une quelconque des revendications 10 à 16, **caractérisé par le fait que** soit les deux empreintes (11a et 11 b) ont une même forme, une même disposition et de mêmes dimensions, constantes et uniformes, d'une extrémité libre à l'autre, soit l'une ou/et l'autre des deux empreintes (11a, 11 b) est légèrement inclinée le long de l'axe (6) du dispositif (1) de pincement de fermeture pour le sectionnement, vers le bord libre de chant de la rive (8a, 8b) et vers l'extrémité proximale (20a, 20b, 21a, 21b), pour exercer un pincement du tube plus important vers l'extrémité proximale (20a, 20b, 21 a, 21 b) et expulser le fluide se trouvant dans le tube vers l'extrémité distale (19).

18. Dispositif (1) de pincement de fermeture pour le sectionnement selon l'une quelconque des revendications 10 à 17, **caractérisé par** la présence de moyens d'écartement constitués par des butées ménagés sur les faces d'extrémité et proximales (20a, 20b, 21 a, 21 b) ou les moyens d'association (24a, 24b).

19. Dispositif (1) de pincement de fermeture pour le sectionnement selon l'une quelconque des revendications 10 à 18, **caractérisé par le fait que** les moyens d'association (24a, 24b, 27, 32) comprennent au moins une saillie (24a) ménagé sur l'une des parties (4a, 4b, 5a, 5b), apte à coopérer de façon amovible avec au moins un trou borgne ou creux ou rainure (24b) complémentaires, ménagé sur l'autre partie de la même section (3a, 3b), et plus particulièrement une saillie (24a) et un trou ou rainure (24b) s'étendant parallèlement à l'axe (6) ou transversalement.

20. Dispositif (1) de pincement de fermeture pour le sectionnement selon l'une quelconque des revendications 10 à 19, **caractérisé par le fait que** soit les moyens d'association (27) comprennent au moins une pièce amovible de liaison en forme de clip (27), apte à associer de façon amovible les deux parties (4a et 4b ou 5a et 5b) de façon indirecte, le clip (27) comprenant une âme (28) de laquelle sont attenants quatre saillies en forme de tige (29a) aptes à coopérer avec quatre rainures (29b) transversales ménagées sur la face extérieure du dispositif (1) de pincement de fermeture pour le sectionnement, sur chaque partie (4a, 4b, 5a, 5b), soit les moyens d'association (32) comprennent une pièce amovible de liaison en forme de berceau (32), apte à associer de façon amovible les deux parties (4a et 4b ou 5a et 5b) de façon indirecte, le berceau (33) comporte deux rives (34) se faisant face, aptes à venir enserrer les parties (4a et 4b ou 5a et 5b) par leurs faces extrêmes distales (19).

21. Dispositif (1) de pincement de fermeture pour le sectionnement selon l'une quelconque des revendications 10 à 20, **caractérisé par le fait que** les deux sections (3a et 3b) sont deux pièces distinctes et sont amenées à l'état fermé par un mouvement relatif de coulissement transversal, l'une (3a) des deux sections (3a et 3b) étant une section mâle et l'autre (3b) étant une section femelle, la section mâle (3a) comportant et étant limitée extérieurement par une première âme (7a) et deux premières rives (8a), la section femelle (3b) comportant et étant limitée extérieurement par une seconde âme (7a) et deux secondes rives (8b).

22. Dispositif (1) de pincement de fermeture pour le sectionnement selon la revendication 21, **caractérisé par le fait que** l'écartement entre les deux faces extérieures (9) opposées des deux premières rives (8a) est en correspondance avec l'écartement entre les deux faces intérieures (10) en regard des deux secondes rives (8b), de manière à permettre, à l'état fermé du dispositif (1) de pincement de fermeture pour le sectionnement, l'emboîtement avec ajustement des deux sections (3a et 3b), la couple de faces extérieures (9) étant alors en contact et en appui sur la couple de faces intérieures (10).

23. Dispositif (1) de pincement de fermeture pour le sectionnement selon l'une quelconque des revendications 10 à 22, **caractérisé par le fait que** les deux sections (3a et 3b) sont deux pièces associées structurellement l'une à l'autre à pivotement autour d'un axe (30) qui, par rapport à l'axe longitudinal (6) du dispositif (1) de pincement de fermeture pour le sectionnement est déporté sur un côté longitudinal du dispositif (1) de pincement de fermeture pour le sectionnement, les deux sections (3a et 3b) étant amenées à l'état fermé par un mouvement relatif de pivotement autour de l'axe de pivotement.(30).

24. Dispositif (1) de pincement de fermeture pour le sectionnement selon l'une quelconque des revendications 10 à 23, **caractérisé par le fait que** les deux sections (3a et 3b) sont de même longueur axiale.

25. Ensemble de pincement de fermeture et de sectionnement (1 + 2) comprenant un dispositif (1) de pincement de fermeture pour le sectionnement selon l'une quelconque des revendications 10 à 24 et au moins un organe de coupe (2) apte à passer dans le passage de sectionnement (22) sans qu'il ne sectionne, ne pénètre dans, ou n'interfère frontalement avec le dispositif (1) de pincement de fermeture pour le sectionnement.

26. Ensemble de pincement de fermeture et de sectionnement (1 + 2) selon la revendication 25, **caractérisé par le fait que** soit le dispositif (1) de pincement de fermeture pour le sectionnement inclut structurellement et fonctionnellement le au moins un organe de coupe (2), plus particulièrement monté coulissant dans son propre plan sur la face d'extrémité proximale (20a, 20b) de l'une des parties (4a, 4b) du dispositif (1) de pincement de fermeture pour le sectionnement soit le dispositif (1) de pincement de fermeture pour le sectionnement n'inclut pas structurellement le au moins un organe de coupe (2), celui-ci lui étant associé fonctionnellement.

## Patentansprüche

1. Verfahren zum Schneiden und Verschließen eines flexiblen Schlauchs, wobei:
- ein zu schneidender und zu verschließender Schlauch vorgesehen ist,
- eine Einheit zum Abklemmen, Verschließen und Schneiden vorgesehen ist, umfassend eine Vorrichtung (1) zum Abklemmen, Verschließen und Schneiden und mindestens ein Schneidorgan (2), wobei die Abklemm-Verschlussvorrichtung (1) zum Schneiden zwei Abschnitte (3a, 3b) umfasst, von denen jeder zwei Hohlteile (4a, 4b und 5a, 5b) aufweist, wobei mindestens ein Paar (4a und 5a oder 4b und 5b) Vertiefungen (11 a, 11 b) aufweist, wobei diese Teile (4a, 4b und 5a, 5b) in einer Linie mit Verbindungsmitteln (24a, 24b, 27, 32) angeordnet sein können und dabei zwischen sich einen quer verlaufenden Durchgang zum Schneiden (22) bilden, der einen Zugang (26) aufweist, der für den Durchgang des Schnittorgans (2) oder eines Steuerorgans des Schnittorgans (2) geeignet ist, wobei sich die Abklemm-Verschlussvorrichtung (1) zum Schneiden entweder im offenen Zustand befinden kann, in dem die beiden Abschnitte (3a, 3b) voneinander beabstandet sind, oder in geschlossenem Zustand, in dem sie nahe beieinander liegen und gegeneinander geschlossen sind und durch Mittel zum Verriegeln (25a, 25b) geschlossen gehalten werden und in dem die Seiten (13, 14) der Vertiefungen (11a, 11 b) einen Durchgang (23) zum Abklemmen und zum Schließen des Schlauchs bilden,
- die Abklemm-Verschlussvorrichtung (1) zum Schneiden in offenem Zustand vorgesehen ist,
- eine Region zum Abklemmen des Schlauchs in der Vertiefung (11a) eines Abschnitts (3a) und der andere Abschnitt (3b) gegenüber dem Vorgenannten angeordnet wird und die Abklemm-Verschlussvorrichtung (1) zum Schneiden mit Zusammenwirken der Verriegelungsmittel (25a, 25b) in den geschlossenen Zustand gebracht wird, bis der Schlauch in mindestens einer Zone zum Verschließen durch positives Abklemmen durch die Seiten (13, 14) positiv abgeklemmt und verschlossen ist und der auf diese Weise abgeklemmte und verschlossene Schlauch zwischen den Abschnitten (3a, 3b) gehalten wird,
- der Schlauch zwischen den Teilen (4a und 5a, 4b und 5b) in geschlossenem Zustand mithilfe des mindestens einen Schneidorgans (2) geschnitten wird,
- die Verbindungsmittel (24a, 24b) und somit die Teile (4a und 4b, 5a und 5b) getrennt werden, wovon das mindestens eine Paar der Teile (4a und 5a oder 4b und 5b) mit Vertiefungen (11 a, 11 b) am geschnittenen Endteil des Schlauchs fixiert bleibt, indem es abgeklemmt wird und indem es verschlossen wird,
**dadurch gekennzeichnet, dass**:
- eine Abklemm-Verschlussvorrichtung (1) zum Schneiden vorgesehen ist, wovon eine der Vertiefungen eine weibliche Vertiefung (11a) ist, die andere der Vertiefungen eine männliche Vertiefung (11 b) ist, wobei die beiden Vertiefungen (11a, 11 b), wenn sich die Abklemm-Verschlussvorrichtung (1) zum Schneiden in geschlossenem Zustand befindet, zwischen sich einen Durchgang zum Verschließen durch positives Abklemmen (23) bilden, der von den beiden Seiten (13, 14) der Vertiefungen (11 a, 11 b) begrenzt ist, die im geraden Querschnitt eine allgemein U-Form aufweisen,
- und, um den Schlauch zu Schneiden, das mindestens eine Schneidorgan (2) in den Durchgang zum Schneiden (22) eingeführt wird, ohne dass das Schneidorgan (2) schneidet, in die Abklemm-Verschlussvorrichtung (1) zum Schneiden eindringt oder diese frontal stört.

2. Verfahren nach Anspruch 1, **dadurch gekennzeichnet, dass** der Schlauch unmittelbar oder eine gewisse Zeit nachdem dessen Verschließen durch positives Abklemmen durchgeführt wurde, geschnitten wird.

3. Verfahren nach einem der Ansprüche 1 und 2, **dadurch gekennzeichnet, dass** das Schneidorgan (2) in den Durchgang zum Schneiden (22) entweder ohne Kontakt mit der Abklemm-Verschlussvorrichtung (1) zum Schneiden oder mit einem gleitenden Oberflächenkontakt mit der Abklemm-Verschlussvorrichtung (1) zum Schneiden eingeführt wird.

4. Verfahren nach einem der Ansprüche 1 bis 3, **dadurch gekennzeichnet, dass** der Schlauch mithilfe eines einzigen Schneidorgans (2) oder mit zwei gegenüberliegenden Schneidorganen (2) geschnitten wird.

5. Verfahren nach einem der Ansprüche 1 bis 4, **dadurch gekennzeichnet, dass** der Schlauch in einer flachgedrückten Zone geschnitten wird, in der der Schlauch nicht positiv abgeklemmt wird.

6. Verfahren nach einem der Ansprüche 1 bis 5, **dadurch gekennzeichnet, dass** ein Abschnitt (3b) gegenüber einem Anderen (3a) und die Abklemm-Verschlussvorrichtung (1) zum Schneiden in geschlossenen Zustand gebracht wird, durch eine relative Quertranslations- oder Schwenkbewegung, um eine Achse (30), die bezogen auf die Längsachse (6) der Abklemm-Verschlussvorrichtung (1) zum Schneiden auf eine Längsseite der Abklemm-Verschlussvorrichtung (1) zum Schneiden verlagert wird.

7. Verfahren nach einem der Ansprüche 1 bis 6, **dadurch gekennzeichnet, dass** der positiv abgeklemmte und verschlossene Schlauch entweder in einer einzigen Zone zum Verschließen durch positives Abklemmen gehalten wird und der Schlauch in der anderen Zone gehalten wird, oder der positiv abgeklemmte und verschlossene Schlauch in zwei Zonen zum Verschließen durch positives Abklemmen gehalten wird.

8. Verfahren nach einem der Ansprüche 1 bis 7, **dadurch gekennzeichnet, dass** die Verbindungsmittel (24a, 24b, 27, 32) und daher die Teile (4a und 4b, 5a und 5b) getrennt werden, während entweder nur das eine der Paare der Teile (4a, 5a und 4b, 5b) mit Vertiefungen (11 a, 11 b) am geschnittenen Endteil des Schlauchs fixiert bleibt, indem es abgeklemmt und verschlossen wird, oder die beiden Paare der Teile (4a, 5a und 4b, 5b) mit Vertiefungen (11 a, 11 b) am geschnittenen Endteil des Schlauchs fixiert bleiben, indem es abklemmt und verschlossen wird.

9. Verfahren nach einem der Ansprüche 1 bis 8, **dadurch gekennzeichnet, dass** das Verschließen durch Abklemmen des Schlauchs in der Zone der beiden seitlichen Falten des Schlauchs begonnen wird.

10. Abklemm-Verschlussvorrichtung zum Schneiden, die insbesondere dazu bestimmt ist, in Kombination mit einem Schneidorgan (2) das Verfahren nach einem der Ansprüche 1 bis 9 durchzuführen, **dadurch gekennzeichnet, dass** sie zwei Abschnitte (3a, 3b) umfasst, wobei Jeder zwei Hohlteile (4a, 4b und 5a, 5b) aufweist, wobei mindestens ein Paar (4a und 5a oder 4b und 5b) Vertiefungen (11 a, 11 b) aufweist, wobei diese Teile (4a, 4b und 5a, 5b) in einer Linie durch Verbindungsmittel (24a, 24b, 27, 32) abnehmbar angeordnet sein können, und dabei zwischen sich einen quer verlaufenden Durchgang zum Schneiden (22) bilden, der einen Zugang (26) aufweist, der für den Durchgang des Schneidorgans (2) oder eines Steuerorgans des Schneidorgans (2) geeignet ist, wobei der quer verlaufende Durchgang zum Schneiden (22) zwischen den beiden Paaren der quer verlaufenden äußersten und proximalen Seiten (20a, 20b, 21 a, 21 b) der Abschnitte (3a, 3b) angeordnet ist, die einander gegenüber und voneinander nahe beabstandet entlang der Achse (6) der Abklemm-Verschlussvorrichtung (1) zum Schneiden liegen, die sich entweder im offenen Zustand befinden kann, in dem die beiden Abschnitte (3a, 3b) voneinander beabstandet sind, oder in geschlossenem Zustand, in dem sie nahe beieinander liegen und gegeneinander geschlossen sind und durch Verriegelungsmittel (25a, 25b) geschlossen gehalten werden und wo die Seiten (13, 14) der Vertiefungen (11a, 11 b) einen Durchgang zum Verschließen durch Abklemmen (23) des Schlauchs bilden, umfassend mindestens ein Stück (23a), dem axial benachbart ein Raum (23b) liegt, in dem der Durchgang (22) mündet und wo der Schlauch einerseits gehalten und andererseits mit einem bestimmten Grad abgeflacht wird, wobei die eine der Vertiefungen (11a) eine weibliche Vertiefung ist, wobei die andere der Vertiefungen eine männliche Vertiefung (11b) ist, wobei die beiden Vertiefungen (11a, 11 b), in geschlossenem Zustand der Abklemm-Verschlussvorrichtung (1) zum Schneiden zwischen sich einen Durchgang zum Verschließen durch positives Abklemmen (23) bilden, der durch die beiden Seiten (13, 14) der Vertiefungen (11a, 11 b) begrenzt ist, der im geraden Querschnitt eine allgemeine U-Form aufweist.

11. Abklemm-Verschlussvorrichtung zum Schneiden nach Anspruch 10, **dadurch gekennzeichnet, dass** eine der Vertiefungen (11a) eine weibliche Vertiefung ist, die andere der Vertiefungen eine männliche Vertiefung (11 b) ist, wobei die beiden Vertiefungen (11 a, 11 b), wenn sich die Abklemm-Verschluss-Vorrichtung (1) zum Schneiden in geschlossenem Zustand befindet, zwischen sich einen Durchgang zum Verschließen durch positives Abklemmen (23) bilden, der von den beiden Seiten (13, 14) der Vertiefungen (11a, 11 b) begrenzt ist, die im geraden Querschnitt eine allgemein U-Form aufweisen.

12. Abklemm-Verschlussvorrichtung zum Schneiden nach einem der Ansprüche 10 und 11, **dadurch gekennzeichnet, dass** die Breite der Öffnung des Durchgangs zum Abklemmen (23), der durch den Querabstand zwischen den Seiten (13 und 14) des geraden Querschnitts mit der allgemeinen U-Form definiert ist, etwas kleiner ist als das Doppelte der Dicke der Wand des abzuklemmenden flexiblen Schlauchs.

13. Abklemm-Verschlussvorrichtung zum Schneiden nach einem der Ansprüche 10 bis 12, **dadurch gekennzeichnet, dass**:
- die männliche Vertiefung (11 b) im geraden Querschnitt eine allgemeine U-Form mit einem Steg und zwei Schenkeln aufweist, die weibliche Vertiefung (11 a) im geraden Querschnitt eine allgemeine U-Form mit einem Steg und zwei Schenkeln aufweist und der Durchgang zum Abklemmen (23) im geraden Querschnitt eine allgemeine U-Form mit einem Steg und zwei Schenkel aufweist,
- die beiden Schenkel gegenüber jedem Schenkelpaar gegenüber der männlichen Vertiefung (11b) und der weiblichen Vertiefung (11a), indem sie sich aneinander annähern, in Richtung ihrer den Stegen der Vertiefungen gegenüberliegenden Enden gehen, mindestens in der Zone dieser Enden, und jeder der beiden Schenkel des Durchgangs zum Abklemmen (23) weist zwischen den Seiten der beiden Vertiefungen (11 a und 11 b) eine Öffnung auf, deren Breite in Richtung ihres dem Steg des Durchgangs zum Abklemmen (23) gegenüberliegenden Endes abnimmt.

14. Abklemm-Verschlussvorrichtung zum Schneiden nach einem der Ansprüche 10 bis 13, **dadurch gekennzeichnet, dass** die beiden Hohlteile (4a, 4b und 5a, 5b) jedes Abschnitts (3a und 3b) analog sind, wobei die Abklemm-Verschlussvorrichtung (1) zum Schneiden eine Funktion zum Schließen des Schlauchs auf sich selbst in zwei Zonen zum Verschließen durch positives Abklemmen gewährleistet, oder verschieden sind, wobei die Abklemm-Verschlussvorrichtung (1) zum Schneiden eine Funktion zum Schließen des Schlauchs auf sich selbst in einer einzigen Zone zum Verschließen durch positives Abklemmen gewährleistet und nur in der anderen Zone gehalten wird.

15. Abklemm-Verschlussvorrichtung zum Schneiden nach einem der Ansprüche 10 bis 14, **dadurch gekennzeichnet, dass** die Mittel zum Verriegeln (25a und 25b) für die beiden Paare der Hohlteile (4a und 5a, 4b und 5b) entweder irreversibel sind oder für eines der Paare der Hohlteile (4a und 5a oder 4b und 5b) irreversibel sind und für das andere (4b und 5b oder 4a und 5a) reversibel sind.

16. Abklemm-Verschlussvorrichtung zum Schneiden nach einem der Ansprüche 10 bis 15, **dadurch gekennzeichnet, dass** der Durchgang zum Abklemmen (23) eine einstellbare Öffnung aufweist, wobei die relative Bewegung, um einen Abschnitt (3b) gegenüber dem anderen (3a) und die Abklemm-Verschlussvorrichtung (1) zum Schneiden in den geschlossenen Zustand zu bringen, mehr oder weniger groß ist, damit der Durchgang zum Abklemmen (23) jeweils mehr oder weniger klein ist.

17. Abklemm-Verschlussvorrichtung zum Schneiden nach einem der Ansprüche 10 bis 16, **dadurch gekennzeichnet, dass** die beiden Vertiefungen (11a und 11 b) eine gleiche Form, eine gleiche Anordnung und die gleichen Abmessungen, die konstant und gleichmäßig von einem freien Ende zum anderen sind, aufweisen, oder die eine und/oder die andere der beiden Vertiefungen (11 a, 11 b) entlang der Achse (6) der Abklemm-Verschlussvorrichtung (1) zum Schneiden in Richtung des freien Rands der Schmalseite (8a, 8b) und zum proximalen Ende (20a, 20b, 21 a, 21 b) leicht geneigt ist, um ein stärkeres Abklemmen des Schlauchs in Richtung des proximalen Endes (20a, 20b, 21 a, 21 b) auszuüben und um das Fluid, das sich in dem Schlauch befindet, in Richtung des distalen Endes (19) auszustoßen.

18. Abklemm-Verschlussvorrichtung zum Schneiden nach einem der Ansprüche 10 bis 17, die durch die Gegenwart von Mitteln zum Beabstanden gekennzeichnet ist, die aus Anschlägen bestehen, die auf den äußersten und proximalen Seiten (20a, 20b, 21 a, 21 b) oder den Verbindungsmitteln (24a, 24b) angebracht sind.

19. Abklemm-Verschlussvorrichtung zum Schneiden nach einem der Ansprüche 10 bis 18, **dadurch gekennzeichnet, dass** die Verbindungsmittel (24a, 24b, 27, 32) mindestens einen Vorsprung (24a) umfassen, der auf einem der Teile (4a, 4b, 5a, 5b) angebracht ist, der geeignet ist, abnehmbar mit mindestens einem komplementären Sackloch oder einer Mulde oder Rille (24b) zusammenzuwirken, das/die auf dem anderen Teil des gleichen Abschnitts (3a, 3b) angeordnet ist, und insbesondere einen Vorsprung (24a) und ein Loch oder eine Rille (24b), die sich parallel zur Achse (6) oder quer dazu erstrecken.

20. Abklemm-Verschlussvorrichtung zum Schneiden nach einem der Ansprüche 10 bis 19, **dadurch gekennzeichnet, dass** die Verbindungsmittel (27) entweder mindestens ein abnehmbares Teil zur Verbindung in Form eines Clips (27) umfassen, das geeignet ist auf abnehmbare Weise die beiden Teile (4a und 4b oder 5a und 5b) auf indirekte Weise zu verbinden, wobei der Clip (27) einen Steg (28) umfasst, an die sich vier Vorsprünge in Form von Stiften (29a) anschließen, die geeignet sind, mit vier quer verlaufenden Rillen (29b) zusammenzuwirken, die auf der Außenseite der Abklemm-Verschlussvorrichtung (1) zum Schneiden auf jedem Teil (4a, 4b, 5a, 5b) angebracht sind, oder die Verbindungsmittel (32) umfassen ein abnehmbares Verbindungsteil in Form eines Rahmen (32), das geeignet ist, die beiden Teile (4a und 4b oder 5a und 5b) auf abnehmbare Weise indirekt zu verbinden, wobei der Rahmen (33) zwei Kanten (34) umfasst, die einander gegenüber liegen, die geeignet sind, die Teile (4a und 4b oder 5a und 5b) durch ihre distalen Endseiten (19) zusammenzuhalten.

21. Abklemm-Verschlussvorrichtung zum Schneiden nach einem der Ansprüche 10 bis 20, **dadurch gekennzeichnet, dass** die beiden Abschnitte (3a und 3b) zwei verschiedene Stücke sind und durch eine relative Verschiebungsbewegung in Querrichtung in geschlossenen Zustand gebracht werden, wobei der Eine (3a) der beiden Abschnitte (3a und 3b) ein männlicher Abschnitt ist und der Andere (3b) ein weiblicher Abschnitt ist, wobei der männliche Abschnitt (3a) einen ersten Steg (7a) und zwei erste Kanten (8a) umfasst und von diesen äußerlich begrenzt ist, wobei der weibliche Abschnitt (3b) einen zweiten Steg (7a) und zwei zweite Kanten (8b) umfasst und von diesen äußerlich begrenzt ist.

22. Abklemm-Verschlussvorrichtung zum Schneiden nach Anspruch 21, **dadurch gekennzeichnet, dass** der Abstand zwischen den beiden Außenseiten (9) gegenüber den beiden ersten Kanten (8a) mit dem Abstand zwischen den beiden Innenseiten (10) gegenüber den beiden zweiten Kanten (8b) übereinstimmt, um, wenn die Abklemm-Verschlussvorrichtung (1) zum Schneiden geschlossen ist, das Ineinandergreifen mit Anpassung der beiden Abschnitte (3a und 3b) zu ermöglichen, wobei das Paar der Außenseiten (9) dann mit dem Paar der Innenseiten (10) in Kontakt ist und zur Anlage kommt.

23. Abklemm-Verschlussvorrichtung zum Schneiden nach einem der Ansprüche 10 bis 22, **dadurch gekennzeichnet, dass** die beiden Abschnitte (3a und 3b) zwei Stücke sind, die schwenkend um eine Achse (30), die bezogen auf die Längsachse (6) der Abklemm-Verschlussvorrichtung (1) zum Schneiden auf eine Längsseite der Abklemm-Verschlussvorrichtung (1) zum Schneiden verlagert ist, strukturell miteinander verbunden sind, wobei die beiden Abschnitte (3a und 3b) durch eine relative Schwenkbewegung um die Schwenkachse (30) in den geschlossenen Zustand gebracht werden.

24. Abklemm-Verschlussvorrichtung zum Schneiden nach einem der Ansprüche 10 bis 23, **dadurch gekennzeichnet, dass** die beiden Abschnitte (3a und 3b) die gleiche Achsenlänge aufweisen.

25. Einheit zum Abklemmen, Verschließen und Schneiden, umfassend eine Abklemm-Verschlussvorrichtung (1) zum Schneiden nach einem der Ansprüche 10 bis 24 und mindestens ein Schneidorgan (2), das geeignet ist, in den Durchgang zum Schneiden (22) zu passen, ohne dass es schneidet, noch in die Abklemm-Verschlussvorrichtung (1) zum Schneiden eindringt oder diese frontal stört.

26. Einheit zum Abklemmen, Verschließen und Schneiden nach Anspruch 25, **dadurch gekennzeichnet, dass** die Abklemm-Verschlussvorrichtung (1) zum Schneiden das mindestens eine Schneidorgan (2) enthält, das entweder strukturell und funktionell, insbesondere gleitend in seiner eigenen Ebene auf der proximalen Außenseite (20a, 20b) von einem der Teile (4a, 4b) der Abklemm-Verschlussvorrichtung (1) zum Schneiden montiert ist, oder die Abklemm-Verschlussvorrichtung (1) zum Schneiden das mindestens eine Schneidorgan (2) nicht strukturell enthält, wobei dieses funktionell mit ihm verbunden ist.

## Claims

1. Process for cutting into sections and closing a flexible tube, in which:
- There is a tube to be cut into sections and closed,
- There is a pinching closing unit for cutting into sections comprising a pinching closing device (1) for cutting into sections and at least one cutting element (2), whereby the pinching closing device (1) for cutting into sections comprises two cross-sections (3a, 3b), each having two hollow parts (4a, 4b and 5a, 5b), at least one pair (4a and 5a or 4b and 5b) having indentations (11a, 11b), whereby these parts (4a, 4b and 5a, 5b) can be placed in the extension of one another by combination means (24a, 24b, 27, 32) while making between them a transverse passage for cutting into sections (22) having an access (26) that can allow the cutting element (2) or a control element of the cutting element (2) to pass, with the pinching closing device (1) for cutting into sections able to be found either in the open state where the two cross-sections (3a, 3b) are separated from one another or in the closed state where they are drawn together and closed on one another and kept closed by locking means (25a, 25b) and where the surfaces (13, 14) of the indentations (11a, 11b) form a passage (23) for pinching and closing the tube,
- There is a pinching closing device (1) for cutting into sections in the open state,
- A pinching region of the tube is positioned in the indentation (11a) of a cross-section (3a), and the other cross-section (3b) is brought opposite the preceding one and the pinching closing device (1) for cutting into sections is brought into the closed state, with cooperation of the locking means (25a, 25b) until the tube is positively pinched and closed in at least one positive pinching closing zone by the surfaces (13, 14), and the tube is held in place thus pinched and closed between the cross-sections (3a, 3b),
- The tube is cut into sections between the parts (4a and 5a, 4b and 5b) in the closed state, using at least one cutting element (2),
- The combination means (24a, 24b) and therefore the parts (4a and 4b, 5a and 5b) are dissociated, of which combination means at least one pair of parts (4a and 5a or 4b and 5b) with indentations (11a, 11b) remains attached to the end part that is cut into sections of the tube by pinching it and by closing it,
**characterized by** the fact that:
- There is a pinching closing device (1) for cutting into sections of which one of the indentations (11a) is a female indentation, and the other indentation is a male indentation (11b), with the two indentations (11a, 11b), in the closed state of the pinching closing device (1) for cutting into sections, making between them a positive pinching closing passage (23) that is limited by the two surfaces (13, 14) of the indentations (11a, 11b), having, in the transverse straight cross-section, a general U shape,
- And, for cutting the tube into sections, at least one cutting element (2) is passed into the passage for cutting into sections (22) without the cutting element (2) cutting into sections, penetrating into, or interfering frontally with the pinching closing device (1) for cutting into sections.

2. Process according to Claim 1, wherein the tube is cut into sections immediately after or a certain time after its positive pinching closing has been implemented.

3. Process according to any of Claims 1 and 2, wherein the cutting element (2) is passed into the passage for cutting into sections (22) either without contact with the pinching closing device (1) for cutting into sections or with a surface contact that slides with the pinching closing device (1) for cutting into sections.

4. Process according to any of Claims 1 to 3, wherein the tube is cut into sections by means of a single cutting element (2) or two opposite cutting elements (2).

5. Process according to any of Claims 1 to 4, wherein the tube is cut into sections in a flattening zone in which the tube is not positively pinched.

6. Process according to any of Claims 1 to 5, wherein one cross-section (3b) is brought opposite another (3a) and the pinching closing device (1) for cutting into sections is brought into the closed state, by a relative movement of transverse translation or pivoting around an axis (30) that, relative to the longitudinal axis (6) of the pinching closing device (1) for cutting into sections, is offset on a longitudinal side of the pinching closing device (1) for cutting into sections.

7. Process according to any of Claims 1 to 6, wherein either the tube is held in place positively pinched and closed in a single positive pinching closing zone, and the tube is held in place in the other zone, or the tube is held in place positively pinched and closed in two positive pinching closing zones.

8. Process according to any of Claims 1 to 7, wherein the combination means (24a, 24b, 27, 32) and therefore the parts (4a and 4b, 5a and 5b) are dissociated, whereas either only one of the pairs of parts (4a, 5a and 4b, 5b) with indentations (11a, 11b) remains attached to the end part that is cut into sections of the tube by pinching it and by closing it, or the two pairs of parts (4a, 5a and 4b, 5b) with indentations (11a, 11b) remain attached to the end part of the tube that is cut into sections by pinching it and by closing it.

9. Process according to any of Claims 1 to 8, wherein the pinching closing of the tube is begun in the zone of two lateral folds of the tube.

10. Pinching closing device (1) for cutting into sections that is specially intended, in combination with a cutting element (2), for the implementation of the process according to any of Claims 1 to 9, wherein it comprises two cross-sections (3a, 3b), each having two hollow parts (4a, 4b, and 5a, 5b), at least one pair (4a and 5a or 4b and 5b) having indentations (11a, 11b), whereby these parts (4a, 4b, and 5a, 5b) can be placed in the extension of one another in a removable way by combination means (24a, 24b, 27, 32) while making between them a transverse passage for cutting into sections (22) that has an access (26) that can allow the cutting element (2) or an element for control of the cutting element (2) to pass, whereby the transverse passage for cutting into sections (22) is made between the two pairs of transverse end surfaces and proximal surfaces (20a, 20b, 21a, 21b) of the cross-sections (3a, 3b), located opposite one another and offset close to one another along the axis (6) of the pinching closing device (1) for cutting into sections, which can be found either in the open state where the two cross-sections (3a, 3b) are separated from one another or in the closed state where they are drawn together and closed on one another and kept closed by locking means (25a, 25b) and where the surfaces (13, 14) of the indentations (11a, 11b) form a passage (23) for pinching and closing the tube, comprising at least one segment (23a) to which a space (23b) is axially adjacent, in which space the passage (22) empties and where the tube is, on the one hand, held in place, and, on the other hand, flattened to a certain degree, with one of the indentations (11a) being a female indentation, and with the other indentation being a male indentation (11b), the two indentations (11a, 11b), in the closed state of the pinching closing device (1) for cutting into sections making between them a positive pinching closing passage (23) that is limited by the two surfaces (13, 14) of the indentations (11a, 11b), having, in a transverse straight cross-section, a general U shape.

11. Pinching closing device (1) for cutting into sections according to Claim 10, wherein one of the indentations (11a) is a female indentation, and the other indentation is a male indentation (11b), whereby the two indentations (11a, 11b) - in the closed state of the pinching closing device (1) for cutting into sections - make between them a positive pinching closing passage (23) that is limited by the two surfaces (13, 14) of the indentations (11a, 11b), having, in the transverse straight cross-section, a general U shape.

12. Pinching closing device (1) for cutting into sections according to any of Claims 10 and 11, wherein the width of the opening of the pinching passage (23), defined by the transverse spacing between the surfaces (13 and 14) of the transverse straight cross-section in general U shape, is slightly smaller than twice the thickness of the wall of the flexible tube to be pinched.

13. Pinching closing device (1) for cutting into sections according to any of Claims 10 to 12, wherein:
- In a transverse straight cross-section, the male indentation (11b) has a general U shape with a core and two wings; in a transverse straight cross-section, the female indentation (11a) has a general U shape with a core and two wings; and in a transverse straight cross-section, the pinching passage (23) has a general U shape with a core and two wings,
- The two wings opposite each pair of wings facing the male indentation (11b) and the female indentation (11a), by drawing together toward their opposite ends, go to the cores of the indentations, at least in the zone of these ends, and each of the two wings of the pinching passage (23) has, between the surfaces of the two indentations (11a and 11b), an opening whose width will diminish towards its end that is opposite to the core of the pinching passage (23).

14. Pinching closing device (1) for cutting into sections according to any of Claims 10 to 13, wherein the two hollow parts (4a, 4b and 5a, 5b) of each cross-section (3a and 3b) are either analogous, with the pinching closing device (1) for cutting into sections ensuring a closing function of the tube on itself in two positive pinching closing zones, or different, with the pinching closing device (1) for cutting into sections ensuring a closing function of the tube on itself in a single positive pinching closing zone and being only held in place in the other zone.

15. Pinching closing device (1) for cutting into sections according to any of Claims 10 to 14, wherein the locking means (25a and 25b) are either irreversible for the two pairs of hollow parts (4a and 5a, 4b and 5b) or irreversible for one of the pairs of hollow parts (4a and 5a or 4b and 5b) and reversible for the other (4b and 5b or 4a and 5a).

16. Pinching closing device (1) for cutting into sections according to any of Claims 10 to 15, wherein the pinching passage (23) has an adjustable opening, with the relative movement for bringing one cross-section (3b) opposite another (3a) and the pinching closing device (1) for cutting into sections in the closed state being more or less large so that the pinching passage (23) is respectively more or less small.

17. Pinching closing device (1) for cutting into sections according to any of Claims 10 to 16, wherein either the two indentations (11a and 11b) have the same shape, the same arrangement and the same dimensions, constant and uniform, from one free end to the next, or one and/or the other of the two indentations (11a, 11b) is slightly inclined along the axis (6) of the pinching closing device (1) for cutting into sections, toward the free border of the edge of the lip (8a, 8b) and toward the proximal end (20a, 20b, 21a, 21b) for exerting a more significant pinching of the tube toward the proximal end (20a, 20b, 21a, 21b) and expelling the fluid that is found in the tube toward the distal end (19).

18. Pinching closing device (1) for cutting into sections according to any of Claims 10 to 17, **characterized by** the presence of separation means that consist of stops made on the end surfaces and proximal surfaces (20a, 20b, 21a, 21b) or combination means (24a, 24b).

19. Pinching closing device (1) for cutting into sections according to any of Claims 10 to 18, wherein the combination means (24a, 24b, 27, 32) comprise at least one projection (24a) made on one of the parts (4a, 4b, 5a, 5b), able to work in a removable way with at least one blind hole or hollow or groove (24b) that is complementary, made on the other part of the same cross-section (3a, 3b), and more particularly a projection (24a) and a hole or groove (24b) extending parallel to the axis (6) or transversely.

20. Pinching closing device (1) for cutting into sections according to any of Claims 10 to 19, wherein either the combination means (27) comprise at least one removable connecting part in the form of a clip (27), able to combine the two parts (4a and 4b or 5a and 5b) in a removable way and in an indirect way, with the clip (27) comprising a core (28) from which four projections that are in rod form (29a) and that are able to work with four transverse grooves (29b) made on the outside surface of the pinching closing device (1) for cutting into sections are adjacent, on each part (4a, 4b, 5a, 5b), or the combination means (32) comprise a removable connecting part in the shape of a cradle (32), able to combine the two parts (4a and 4b or 5a and 5b) in a removable way and an indirect way; the cradle (33) comprises two lips (34) that face one another, able to encircle the parts (4a and 4b or 5a and 5b) by their distal end surfaces (19).

21. Pinching closing device (1) for cutting into sections according to any of Claims 10 to 20, wherein the two cross-sections (3a and 3b) are two separate parts and are brought to the closed state by a relative transverse sliding movement, whereby one (3a) of the two cross-sections (3a and 3b) is a male cross-section and the other (3b) is a female cross-section, with the male cross-section (3a) comprising and being limited on the outside by a first core (7a) and two first lips (8a), and with the female cross-section (3b) comprising and being limited on the outside by a second core (7a) and two second lips (8b).

22. Pinching closing device (1) for cutting into sections according to Claim 21, wherein the separation between the two opposite outside surfaces (9) of the first two lips (8a) corresponds to the separation between the two inside surfaces (10) opposite the second two lips (8b), in such a way as to allow, in the closed state of the pinching closing device (1) for cutting into sections, the interlocking with adjustment of the two cross-sections (3a and 3b), with the pair of outside surfaces (9) then being in contact and supported on the pair of inside surfaces (10).

23. Pinching closing device (1) for cutting into sections according to any of Claims 10 to 22, wherein the two cross-sections (3a and 3b) are two parts that are structurally combined with one another in pivoting around an axis (30) that, relative to the longitudinal axis (6) of the pinching closing device (1) for cutting into sections, is offset on a longitudinal side of the pinching closing device (1) for cutting into sections, with the two cross-sections (3a and 3b) being brought into the closed state by a relative pivoting movement around the pivoting axis (30).

24. Pinching closing device (1) for cutting into sections according to any of Claims 10 to 23, wherein the two cross-sections (3a and 3b) have the same axial length.

25. Pinching closing unit for cutting into sections (1 + 2) comprising a pinching closing device (1) for cutting into sections according to any of Claims 10 to 24 and at least one cutting element (2) that is able to pass into the passage for cutting into sections (22) without said cutting element cutting into sections, penetrating into, or interfering frontally with the pinching closing device (1) for cutting into sections.

26. Pinching closing unit for cutting into sections (1 + 2) according to Claim 25, wherein either the pinching closing device (1) for cutting into sections structurally and functionally includes the at least one cutting element (2), more particularly mounted to slide in its own plane on the proximal end surface (20a, 20b) of one of the parts (4a, 4b) of the pinching closing device (1) for cutting into sections, or the pinching closing device (1) for cutting into sections structurally does not include the at least one cutting element (2), the latter being combined with it functionally.
